(19) Europäisches Patentamt
European Patent Office
Office européen des brevets

(11) EP 3 233 996 B1

(12) FASCICULE DE BREVET EUROPEEN

(45) Date de publication et mention de la délivrance du brevet:
**03.04.2019 Bulletin 2019/14**

(21) Numéro de dépôt: **15825842.6**

(22) Date de dépôt: **16.12.2015**

(51) Int Cl.:
***C08J 11/18*** *(2006.01)* ***C07C 1/213*** *(2006.01)*
***C07C 1/22*** *(2006.01)* ***C07C 29/09*** *(2006.01)*

(86) Numéro de dépôt international:
**PCT/IB2015/059684**

(87) Numéro de publication internationale:
**WO 2016/098021 (23.06.2016 Gazette 2016/25)**

# (54) PROCÉDÉ DE DÉPOLYMÉRISATION DE MATÉRIAUX POLYMÈRES OXYGÉNÉS

VERFAHREN ZUR DEPOLYMERISIERUNG VON OXYGENIERTEN POLYMERMATERIALIEN

METHOD FOR DEPOLYMERISING OXYGENATED POLYMER MATERIALS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.12.2014 FR 1462581**

(43) Date de publication de la demande:
**25.10.2017 Bulletin 2017/43**

(73) Titulaire: **Commissariat à l'énergie atomique et aux énergies alternatives**
**75015 Paris (FR)**

(72) Inventeurs:
- **CANTAT, Thibault**
  **92130 Issy-les-Moulineaux (FR)**
- **FEGHALI, Elias**
  **Springfield**
  **NZ-3015 ROTURUA (NZ)**

(74) Mandataire: **Gevers & Orès**
**41 avenue de Friedland**
**75008 Paris (FR)**

(56) Documents cités:
- **ERIC M. KRALL ET AL: "Controlled hydrogenative depolymerization of polyesters and polycarbonates catalyzed by ruthenium(ii) PNN pincer complexes", CHEMICAL COMMUNICATIONS, vol. 50, no. 38, 20 mars 2014 (2014-03-20) , page 4884, XP055206893, ISSN: 1359-7345, DOI: 10.1039/c4cc00541d**
- **CÁTIA SANTOS NUNES ET AL: "PET depolymerisation in supercritical ethanol catalysed by [Bmim][BF4]", RSC ADVANCES, vol. 4, no. 39, 20 mars 2014 (2014-03-20), page 20308, XP055210283, ISSN: 2046-2069, DOI: 10.1039/c4ra00262h**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne un procédé de dépolymérisation de matériaux polymères oxygénés. La présente invention concerne aussi un procédé de recyclage de matériaux plastiques ou des mélanges de matériaux plastiques contenant au moins un polymère oxygéné et aussi un procédé de préparation des composés aromatiques mono-, di-, et/ou tri-cycliques dont chaque cycle est éventuellement mono-, di- et /ou tri-oxygénés caractérisé en ce qu'il comprend une étape de dépolymérisation de matériaux polymères oxygénés.

Selon la présente invention un polymère oxygéné désigne un polymère ou copolymère dont les motifs de répétition de la chaîne principale contiennent la fonction ester (-CO-O-) appelés également les polyesters ou la fonction carbonate (-O-CO-O-) appelés également les polycarbonates.

Les composés aromatiques obtenus par le procédé de dépolymérisation de matériaux polymères oxygénés selon l'invention, peuvent être utilisés pour la fabrication de combustibles, de composants électroniques, de polymères plastiques, de caoutchouc, de médicaments, de vitamines, de produits cosmétiques, de parfums, de produits alimentaires, de fils et fibres synthétiques, de cuirs synthétiques, de colles, de pesticides, d'engrais.

Les polymères oxygénés sont actuellement des constituants de base d'une grande partie des matériaux de la vie courante et en particulier des plastiques. En effet, un matériau plastique est essentiellement constitué d'un polymère qui après des opérations de moulage et de façonnage, conduit à l'obtention d'un objet fini ou semi-fini. Ces plastiques ont généralement des masses moléculaires élevées et sont souvent issus de la pétrochimie, mais il existe des plastiques d'origine naturelle. De nos jours, un intérêt croissant envers les matériaux plastiques est observé en raison de leur facilité de fabrication, de leur coût relativement faible ainsi qu'à la polyvalence qu'ils présentent. Cependant, le coût relativement élevé du recyclage de ces matériaux en utilisant les procédés actuels pose des problèmes d'ordre économiques nécessitant de nouvelles solutions pouvant s'accorder avec l'évolution des législations. Ainsi, le recyclage des matériaux contenant des polymères oxygénés est devenu un enjeu important de la société contemporaine.

Plusieurs méthodes de recyclage ont été développées pour faire face à cette problématique. Parmi ces méthodes, le recyclage chimique (ou recyclage tertiaire) est une méthode de recyclage qui est en accord avec les principes du développement durable. En effet, ce type de recyclage permet de récupérer des constituants de la pétrochimie, des déchets des matériaux polymères et des déchets plastiques et de les utiliser comme précurseurs dans la création de produits à hautes valeurs ajoutées. Les matériaux polymères peuvent ainsi être considérés comme une source de matières carbonées (S.M. Al-Salem, P. Lettieri, J. Baeyens, Progress in Energy and Combustion Science, 2010, 36 (2010) pages 103-129 ; S. H. Park and S. H. Kim, Fashion and Textiles, 2014, 1, pages 1-17).

Les méthodes de recyclage chimiques sont généralement divisées en deux catégories : celles qui régénèrent les monomères ou des oligomères de départ et celles qui génèrent d'autres types de molécules ayant des applications en chimie fine ou comme carburant. De nombreuses méthodes de recyclage chimiques existent dans la littérature (D. S. Achilias, D. A. Louka, G. Tsintzou, I. A. Koutsidis, I. Tsagkalias, L. Andriotis, N. P. Nianias, P. Siafaka, Recent Advances in the Chemical Recycling of Polymers (PP, PS, LDPE, HDPE, PVC, PC, Nylon, PMMA), INTECH Open Access Publisher, 2012, ISBN: 953510327X, 9789535103271, E.M Krall, et al., Controlled hydrogenative depolymerization of polyesters and polycarbonates catalyzed by ruthenium(ii) PNN pincer complexes, CHEMICAL COMMUNICATIONS, vol. 50, no. 38, 1st January 2014, pages 4863-4960; C. S. Nunes, et al., PET depolymerisation in supercritical ethanol catalysed by [Bmim][BF4], RSC ADVANCES, vol. 4, no. 39, 15th April 2014, pages 20308-20316).

Toutefois, les méthodes de recyclage chimique ou recyclage tertiaire présentent des inconvénients opérationnels non négligeables comme la conduite de la réaction à haute température et à hautes pressions ainsi que l'utilisation des métaux pour catalyser les réactions. De plus, rares sont les méthodes permettant de recycler à la fois plusieurs types de résines polymères (recyclage de copolymères ou de mélange de polymères) et qui résistent aux additifs présents dans les matériaux.

Ainsi, il existe un réel besoin de développer une méthode alternative aux méthodes de recyclage tertiaire des matériaux polymères déjà existantes, en particulier des matériaux polymères oxygénés, en composés ayant une haute valeur ajoutée palliant les inconvénients des méthodes de recyclage tertiaire de l'art antérieur.

En particulier, il existe donc un réel besoin de développer un procédé de dépolymérisation pouvant être appliqué au recyclage des matériaux polymères, en particulier des matériaux polymères oxygénés, en composés ayant une haute valeur ajoutée.

Plus particulièrement, il existe un réel besoin d'un procédé de dépolymérisation de matériaux polymères, en particulier les matériaux polymères oxygénés :

- qui conduise à la formation de composés moins oxygénés ayant un contenu énergétique plus important ;
- respectueux de l'environnement ;
- pouvant être mis en oeuvre dans des conditions opératoires douces et industriellement intéressantes ;
- évitant l'utilisation de catalyseurs à base de métaux polluants et coûteux ;
- qui soit efficace, l'efficacité se traduisant par une bonne conversion voire une conversion totale du matériau polymère

oxygéné en composés chimiques avec une bonne pureté (au moins 90 % molaire par rapport au nombre total de mole de composés obtenus) ou pouvant être facilement purifiés ;

- avec une bonne sélectivité par rapport aux composés chimiques obtenus ;
- ayant une sélectivité qui puisse être modulée et adaptée en fonction de(s) composé(s) chimique(s) que l'on cherche à préparer ;
- permettant un clivage sélectif de certaines liaisons du matériau polymère oxygéné ;
- qui soit général et versatile pouvant s'adapter au type du matériau polymère oxygéné à dépolymériser ; et/ou
- capable de résister aux additifs éventuellement présents dans les matériaux polymères oxygénés à dépolymériser.

La présente invention a précisément pour but de répondre au moins à ces besoins en fournissant un procédé de dépolymérisation de matériaux polymères oxygénés par clivage sélectif des liaisons oxygène-carbonyle des fonctions esters (-CO-O-) et des fonctions carbonates (-O-CO-O-), caractérisé en ce qu'il comprend une étape de mise contact desdits matériaux polymères oxygénés avec un composé silane de formule (I)

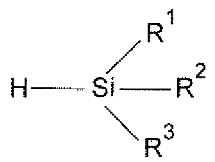

(I)

dans laquelle

- $R^1$, $R^2$ et $R^3$ représentent, indépendamment l'un de l'autre, un atome d'hydrogène, un atome d'halogène, un groupe hydroxyle, un groupe alkyle, un groupe alcényle, un groupe alcynyle, un groupe alkoxy, un groupe aryloxy, un groupe silylé, un groupe siloxy, un groupe aryle, un groupe amino, lesdits groupes alkyle, alcényle, alcynyle, alkoxy, silylé, siloxy, aryle et amino étant éventuellement substitués, ou
- $R^1$ est tel que défini ci-dessus et $R^2$ et $R^3$, pris ensemble avec l'atome de silicium auquel ils sont liés forment un hétérocycle silylé éventuellement substitué ; en présence d'un catalyseur comme décrit dans la revendication 1.

Au sens de l'invention, un matériau polymère oxygéné est un matériau comprenant au moins un polymère oxygéné et éventuellement au moins un additif. La quantité de polymère(s) oxygéné(s) ainsi que la quantité et la nature des additifs présents dans le matériau polymère oxygéné varient en fonction du matériau polymère et des applications visées. Toutefois, dans le cadre de l'invention, au moins 50% en masse du matériau polymère oxygéné est du polymère oxygéné.

Dans le contexte de la présente invention, un polymère oxygéné désigne un polymère ou copolymère dont les motifs de répétition de la chaîne principale contiennent la fonction ester (-CO-O-) appelés également les polyesters ou la fonction carbonate (-O-CO-O-) appelés également les polycarbonates.

Les polymères oxygénés de l'invention sont principalement des polymères synthétiques ou semi-synthétiques mais peuvent aussi être des polymères biosourcés c'est-à-dire issus de la biomasse animale ou végétale. Le ou les additifs éventuellement présents dans le matériau peuvent être introduits avant ou pendant la mise en forme du matériau, pour apporter ou améliorer une (ou parfois plusieurs) propriété(s) spécifique(s). A titre d'exemple d'additifs on peut citer les stabilisants, les antioxydants, les colorants, les pigments, les agents mouillants, les dispersants, les émulsifiants, les épaississants, les biocides, les plastifiants, les photoprotecteurs, etc.

Le procédé de l'invention a l'avantage de résister à la présence d'additif(s) dans les matériaux polymères. Aucun problème d'empoisonnement de catalyseur n'a pu être observé avec les additifs couramment utilisés dans les matériaux polymères. En effet, le plus grand défi du recyclage ne se limite pas à la dépolymérisation du polymère présent seul dans le milieu réactionnel (polymère pur) mais s'étend également à sa dépolymérisation dans un matériau commercial pouvant contenir des additifs comme des colorants, des charges minérales, des antioxydants, etc. La présence de ces additifs dans le matériau peut désactiver le catalyseur employé pour réaliser la dépolymérisation, et ainsi rendre la réaction inefficace. Le procédé de l'invention présente donc un grand intérêt industriel car elle est capable de résister aux additifs et/ou impuretés présents dans le matériau polymère de départ, qui, par exemple, peut être un déchet plastique.

Le procédé de dépolymérisation des matériaux polymères oxygénés selon l'invention est schématiquement représenté en Figure 1.

Les matériaux polymères oxygénés de l'invention peuvent être un polymère oxygéné, ou un mélange de polymères oxygénés, ou un mélange d'au moins deux polymères dont l'un au moins est un polymère oxygéné au sens de l'invention, avec éventuellement un ou plusieurs additif(s).

De préférence, les matériaux oxygénés de l'invention comprennent un ou plusieurs additif(s).

Lorsque le polymère oxygéné est un copolymère, la chaîne principale dudit copolymère peut comporter des motifs de répétition contenant une ou plusieurs fonctions ester (-CO-O-) et/ou une ou plusieurs fonctions carbonate (-O-CO-O-) et éventuellement des motifs de répétition choisis parmi des motifs éthylénique, propylénique, vinylique, vinylique substitué par un ou plusieurs atomes de chlore ou de fluor, styrénique, styrène-butadiène, acrylique, méthacrylique.

Dans la suite de l'exposé, le terme « polymère » peut également désigner un « copolymère ». Ainsi, le terme polymère peut couvrir les homopolymères (un polymère issu d'une seule espèce de monomère) et les copolymères (un polymère issu d'au moins deux monomères différents).

Les polymères oxygénés synthétiques ou semi-synthétiques de l'invention peuvent être choisis parmi :

- les polyesters saturés ou insaturés choisis, par exemple, parmi l'acide polyglycolic (PGA), l'acide polylactique (PLA), le polycaprolactone (PCL), le polyhydroxyalkanoate (PHA), le polyhydroxybutyrate (P3HB), le polyhydroxyvalérate (PHV), le polyéthylène adipate (PEA), le polybutylène succinate (PBS), le poly(3-hydroxybutyrate-*co*-3-hydroxyvalérate (PHBV), le polyéthylène téréphthalate (PET), le polybutylène téréphthalate (PBT), le polytriméthylène téréphthalate (PTT), le polyéthylène naphthalate (PEN),
- les polycarbonates choisis, par exemple, parmi le PC-BPA, le polypropylène carbonate (PPC), le polyéthylène carbonate (PEC), le poly(hexaméthylène carbonate), l'allyl diglycol carbonate (ADC) ou CR-39.

Parmi les polymères oxygénés, le polyéthylène téréphtalate (PET), l'acide polylactique (PLA) et le polycarbonate (PC-BPA) sont les plus étudiés dans la littérature car leur recyclage présente plusieurs intérêts :

➢ Le polytéréphtalate d'éthylène (PET) est l'un des plastiques les plus couramment utilisés dans le monde du fait de sa légèreté, de sa durabilité, de sa résistance chimique mais aussi de son faible coût.

➢ L'acide polylactique (PLA) est très intéressant d'un point de vue environnemental puisqu'il est principalement dérivé de ressources renouvelables, telles que le maïs, les pommes de terre et d'autres produits agricoles (mais compétition avec l'alimentaire ici). En raison de sa biodegrabilité combinée à sa résistance mécanique et sa transparence, le PLA est considéré comme un matériau vert et durable qui peut être vue comme une alternative prometteuse aux résines polymères à base de pétrole notamment le PET.

➢ Les polycarbonates (PC-BPA) sont des thermoplastiques ayant d'excellentes propriétés mécaniques et une grande résistance aux chocs, une résistance aux UV, ainsi qu'une excellente résistance électrique. En conséquence, les polycarbonates sont utilisés dans une grande variété d'applications comme dans les disques compacts, les fenêtres blindées, les emballages alimentaires ou les bouteilles de boissons gazeuses.

Dans le cadre de l'invention, les polymères oxygénés biosourcés sont plus particulièrement ceux issus de la biomasse végétale dont les motifs aromatiques sont liés par des liaisons esters. A ce titre, on peut citer les tannins hydrolysables notamment les gallotannins et les ellagitannins, et la subérine.

Le procédé de dépolymérisation de l'invention pour quelques matériaux polymères oxygénés est représenté en Figure 2.

Les polymères oxygénés sont avantageusement choisis parmi

- les polyesters notamment le PET et le PLA ;
- les polycarbonates notamment le PC-BPA ; et/ou
- les tannins hydrolysables notamment les gallotannins et les ellagitannins, et la subérine.

Comme déjà indiqué, les matériaux de l'invention peuvent être un mélange d'au moins deux polymères dont l'un au moins est un polymère oxygéné au sens de l'invention. Dans ce cas, le ou les autres polymère(s) présent(s) dans le matériau peu(ven)t être choisi(s) parmi les polyoléfines notamment le polyéthylène et le polypropylène ; le polyacétate de vinyle (PVAC), l'alcool polyvinylique (PVAL) ; le polystyrène (PS), l'acrylonitrile butadiène styrène (ABS) ; le styrène-butadiène (SBR) ; l'acrylonitrile styrène acrylate (ASA) ; les polyuréthanes saturés ou réticulés ; le polyméthacrylate de méthyle (PMMA), le polyacrylonitrile (PAN) ; le polychlorure de vinyle (PVC), le polychlorure de vinylidène (PVDC) ; le polytétrafluoroéthylène (PTFE), le polyfluorure de vinyle (PVF), le polyfluorure de vinylidène (PVDF), l'éthylène tétrafluoroéthylène (ETFE), le perfluoroalkoxy (PFA) ; le polyétheréthercétone (PEEK) ; les copolymères séquencés styrène-butadiène-styrène (SBS) ; les polyamides comme le PA6, le PA 12, le PA 6.6 ; les polyuréthanes ; les polyurées ; les copolymères de polyurées et de polyuréthanes (connus sous les nom de Spandex, Lycra ou elastane).

Le procédé de dépolymérisation des matériaux polymères oxygénés selon l'invention fournit des composés chimiques pouvant contenir des groupes siloxy et ayant un nombre de carbones plus réduit que celui du ou des polymères oxygénés présent(s) dans le matériau de départ. Les composés obtenus peuvent conduire, après hydrolyse, à des

composés chimiques de masse molaire moyenne inférieure à 600 g/mol.

L'hydrolyse des composés chimiques contenant des groupes siloxy issus de la dépolymérisation, peut conduire aux composés hydroxylés correspondants qui, à leur tour, peuvent conduire aux composés désoxygénés correspondants en employant les conditions d'hydrosilylation réductrice décrites dans ce procédé.

Le procédé de dépolymérisation selon l'invention peut conduire à la formation de composés chimiques à l'état gazeux comme le méthane, l'éthane, le propane, à l'état liquide comme le paraxylène, l'éthylène glycol et/ou à l'état solide comme le bisphénol A (BPA).

Dans le cas de la dépolymérisation de polymères oxygénés naturels, les produits de dépolymérisation peuvent être des composés aromatiques monocycliques (cas de la gallotannin par exemple), mono- bi- et/ou tri-cycliques (cas de l'ellagitannin par exemple) et éventuellement mono-, di-, et/ou tri-oxygénés. La dépolymérisation peut également générer des molécules non aromatiques, saturés ou insaturés, constitués d'atomes de carbone et d'hydrogène pouvant être éventuellement mono-, di-, et/ou tri-oxygénés.

En fonction de la nature du matériau de départ utilisé, la nature de l'hydrosilane et sa quantité, le catalyseur employé et la durée de la réaction, la nature du produit de dépolymérisation peut changer. Ainsi, en variant les conditions opératoires mises en oeuvre, un type de produit peut être sélectivement obtenu.

En effet, l'un des avantages du procédé de l'invention est la possibilité d'obtenir sélectivement une variété de composés chimiques à partir d'un même type de polymère en contrôlant les conditions opératoires. Par exemple, dans le cas du PET, le procédé de dépolymérisation peut conduire à l'obtention du 1,4-phénylène diméthanol et de l'éthylène glycol, ou bien de l'éthane et du paraxylène.

Dans le cadre de la présente invention, la sélectivité se rapporte à la nature des produits formés ainsi qu'à la nature des liaisons clivées.

Les liaisons visées et clivées sélectivement par le procédé de dépolymérisation de l'invention sont les liaisons oxygène-carbonyle des fonctions esters (-CO-O-) et des fonctions carbonates (-O-CO-O). Ainsi, les liaisons C-O des fonctionnalités dans lesquelles l'atome de carbone est lié à un autre atome de carbone par une liaison multiple sp ou $sp^2$ (par exemple C=C-O) ne sont pas clivées lors du procédé de dépolymérisation de l'invention. Par exemple, les aryles éthers présents dans les polyphénols ne sont pas clivés. Les liaisons C-C simples, doubles et triples ne sont pas non plus clivées par le procédé de dépolymérisation de l'invention. Par exemple le polystyrène (PS) et le chlorure de polyvinyle (PVC) ne sont pas dépolymérisés par le procédé de l'invention.

Selon les conditions opératoires, pendant le procédé de dépolymérisation, la fonction carbonyle -C=O peut être réduite en un éther silylé $-CH-OSiR^1R^2R^3$ où $R^1$, $R^2$, et $R^3$ sont tels que définis dans le cadre de la présente invention. Les conditions opératoires peuvent également être choisies de manière à poursuivre la réduction de l'éther silylé par rupture de la liaison C-O de ce dernier, afin de désoxygéner davantage la molécule en une fonction méthylénique $-CH_2-$.

Le procédé de dépolymérisation de l'invention est d'une grande versatilité notamment par rapport aux types de matériaux polymères oxygénés de départ.

Par ailleurs, en contrôlant minutieusement les conditions opératoires, la dépolymérisation d'un matériau polymère comprenant un mélange de polyester(s) et/ou de polycarbonate(s) peut être sélective. Sans vouloir être lié par la théorie, la différence de réactivité des liaisons oxygène-carbonyle d'une fonction ester et d'une fonction carbonate peut favoriser le clivage sélective de l'une de ces fonctions par rapport à l'autre. De plus, l'effet électronique (par exemple, les effets inductifs liés à la polarisation d'une liaison $\sigma$ et les effets mésomères dus à la délocalisation des électrons $\pi$) et l'encombrement stérique des substituants à proximité de la liaison oxygène-carbonyle peuvent avoir un impact sur la réactivité de la liaison à cliver. Par exemple, dans le cas d'un matériau comprenant un mélange PC-BPA + PLA ou d'un matériau comprenant un mélange PET + PLA, le PC-BPA et le PET sont sélectivement clivés.

D'autre part, l'étape de dépolymérisation dans le procédé de l'invention est réalisée dans des conditions opératoires douces (*i.e.* basses températures et pressions) et permet de s'affranchir des conditions réactionnelles drastiques de température et de pression utilisées traditionnellement, par exemple, dans le recyclage des matériaux polymères.

Par ailleurs, le rendement en composés chimiques de masse molaire moyenne inférieure à 600 g/mol obtenus par le procédé de dépolymérisation et après l'étape d'hydrolyse, dépend du matériau polymère de départ ainsi que des conditions opératoires appliquées. Le rendement est en général bon (de 30 à 99 % molaire par rapport au nombre total de moles de motifs monomères présents dans le(s) polymère(s) du matériau de départ. Par approximation, et afin de calculer le rendement molaire du procédé de dépolymérisation, le matériau polymère de départ est considéré être exclusivement formé du polymère étudié.

Le rendement est calculé en appliquant la formule suivante :

$$\text{Rendement} = \text{n (molécules)} / \text{n (motifs monomères)}$$

n (molécules) étant le nombre de mole d'une molécule donnée de masse molaire moyenne inférieure à 600 g/mol)

obtenu après dépolymérisation et
n (motifs monomères) étant le nombre de mole totale de motifs monomères présents dans les polymères initialement introduits.

Il est à noter que le nombre de motifs monomères est diffèrent du nombre de liaisons pouvant être clivées. La quantification se fait par rapport au nombre de moles de motifs dans le polymère qui peuvent potentiellement donner des molécules simples (de masse molaire moyenne inférieure à 600 g/mol). Après la dépolymérisation ces motifs sont nommés « motifs monomères présents dans les polymères ».

On entend par « alkyle », au sens de la présente invention, un radical carboné linéaire, ramifié ou cyclique, saturé, éventuellement substitué, comprenant 1 à 12 atomes de carbone, de préférence 1 à 8 atomes de carbone. A titre d'alkyle saturé, linéaire ou ramifié, on peut citer par exemple les radicaux méthyle, éthyle, propyle, butyle, pentyle, hexyle, octyle, nonyle, décyle, undécyle, dodécanyle et leurs isomères ramifiés. Comme alkyle cylique, on peut citer les radicaux cyclopropyle, cyclobutyle, cyclopentyle, cyclohexyle, bicylco[2,1,1] hexyle, bicyclo[2,2,1] heptyle. Comme alkyles cycliques portant une insaturation, on peut citer par exemple le cyclopentényle, le cyclohexényle.

Par « alcényle » ou « alcynyle », on entend un radical carboné insaturé linéaire, ramifié ou cyclique, éventuellement substitué, ledit radical carboné insaturé comprenant 2 à 12 atomes de carbone, de préférence 2 à 8 atomes de carbone, comprenant au moins une double liaison (alcényle) ou une triple liaison (alcynyle). A ce titre, on peut citer, par exemple, les radicaux éthylényle, propylényle, butényle, pentényle, hexényle, acétylényle, propynyle, butynyle, pentynyle, hexynyle et leurs isomères ramifiés.

Les groupes alkyle, alcényle et alcynyle, au sens de l'invention, peuvent être éventuellement substitués par un ou plusieurs groupes hydroxyle ; un ou plusieurs groupes alkoxy ; un ou plusieurs groupes siloxy ; un ou plusieurs atomes d'halogène choisis parmi les atomes de fluor, chlore, brome et iode ; un ou plusieurs groupes nitro ($-NO_2$) ; un ou plusieurs groupes nitrile (-CN) ; un ou plusieurs groupes aryle ; avec les groupes alkoxy, <u>siloxy</u> et aryle tels que définis dans le cadre de la présente invention.

Le terme « aryle » désigne de manière générale un substituant aromatique cyclique comportant de 6 à 20, de préférence de 6 à 12 atomes de carbone. Dans le cadre de l'invention le groupe aryle peut être mono- ou polycyclique. A titre indicatif, on peut citer les groupes phényle, benzyle et naphtyle. Le groupe aryle peut être éventuellement substitué par un ou plusieurs groupes hydroxyle, un ou plusieurs groupes alkoxy, un ou plusieurs groupes siloxy, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes alkyle, avec les groupes alkoxy, siloxy et alkyle tels que définis dans le cadre de la présente invention.

Le terme « hétéroaryle » désigne de manière générale un substituant aromatique mono- ou polycyclique comportant de 5 à 10 membres, de préférence de 5 à 7 membres dont au moins 2 atomes de carbone, et au moins un hétéroatome choisi parmi l'azote, l'oxygène, le bore, le silicium, le phosphore et le soufre. Le groupe hétéroaryle peut être mono- ou polycyclique. A titre indicatif, on peut citer les groupes furyle, benzofuranyle, pyrrolyle, indolyle, isoindolyle, azaindolyle, thiophényle, benzothiophényle, pyridyle, quinolinyle, isoquinolyle, imidazolyle, benzimidazolyle, pyrazolyle, oxazolyle, isoxazolyle, benzoxazolyle, thiazolyle, benzothiazolyle, isothiazolyle, pyridazinyle, pyrimidilyle, pyrazinyle, triazinyle, cinnolinyle, phtalazinyle, quinazolinyle. Le groupe hétéroaryle peut être éventuellement substitué par un ou plusieurs groupes hydroxyle, un ou plusieurs groupes alkoxy, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes aryle, un ou plusieurs groupes alkyle, avec les groupes alkyle, alkoxy et aryle tels que définis dans le cadre de la présente invention.

Le terme « alkoxy » signifie un groupe alkyle, alcényle et alcynyle, tels que définis ci-dessus, lié par un atome d'oxygène (-O-alkyle, O-alcényle, O-alcynyle).

Le terme « aryloxy » signifie un groupe aryle tel que défini ci-dessus, lié par un atome d'oxygène (-O-aryle).

Le terme « hétérocycle » désigne de manière générale un substituant mono- ou polycyclique, comportant de 5 à 10 membres, de préférence de 5 à 7 membres, saturé ou instauré, contenant de 1 à 4 hétéroatomes choisis indépendamment l'un de l'autre, parmi l'azote, l'oxygène, le bore, le silicium, le phosphore et le soufre. A titre indicatif, on peut citer les substituants morpholinyle, pipéridinyle, pipérazinyle, pyrrolidinyle, imidazolidinyle, imidazolinyle, pyrazolidinyle, tétrahydrofuranyle, tétrahydropyranyle, thianyle, oxazolidinyle, isoxazolidinyle, thiazolidinyle, isothiazolidinyle. L'hétérocycle peut être éventuellement substitué par un ou plusieurs groupes hydroxyle, un ou plusieurs groupes alkoxy, un ou plusieurs groupes aryle, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes alkyle, avec les groupes alkyle, alkoxy et aryle tels que définis dans le cadre de la présente invention.

Par atome d'halogène, on entend un atome choisi parmi les atomes de fluor, chlore, brome et iode.

Par groupe « silylé », on entend un groupe de formule $[-Si(X)_3]$ dans lequel chaque X, indépendamment l'un de l'autre, est choisi parmi un atome d'hydrogène ; un ou plusieurs atomes d'halogène choisis parmi les atomes de fluor, chlore, brome ou iode ; un ou plusieurs groupes alkyle ; un ou plusieurs groupes alkoxy ; un ou plusieurs groupes

aryle ; un ou plusieurs groupes siloxy ; avec les groupes alkyle, alkoxy, aryle et siloxy tels que définis dans le cadre de la présente invention. Lorsque l'un au moins des X représente plusieurs groupes siloxy, lesdits groupes siloxy peuvent se répéter plusieurs fois de manière à conduire à des organosilanes polymériques de formule générale

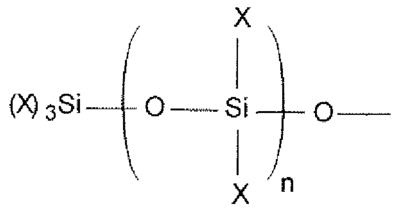

dans laquelle X est tel que défini ci-dessus et n est un nombre entier compris entre 1 et 20000, avantageusement entre 1 et 5000, plus avantageusement entre 1 et 1000. A ce titre on peut citer, par exemple, les radicaux monovalents du polydiméthylsiloxane (PDMS), du polyméthylhydroxysiloxane (PMHS) et du tétraméthyldisiloxane (TMDS) comme représentés ci-dessous par des formules semi développées :

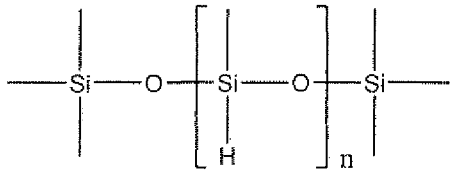

PMHS

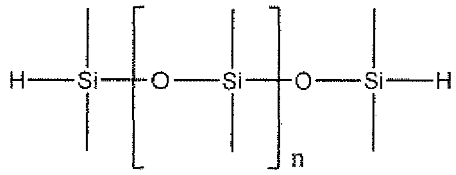

PDMS

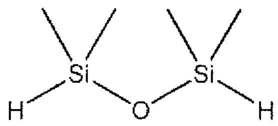

TMDS

Par groupe « siloxy », on entend un groupe silylé, tel que défini ci-dessus, lié par un atome d'oxygène ($-O-Si(X)_3$) avec X tel que défini ci-dessus.

Au sens de l'invention, par « hétérocycle silylé », on entend un substituant mono- ou polycyclique, comportant de 5 à 15 membres, de préférence de 5 à 7 membres, saturé ou insaturé, contenant au moins un atome de silicium et éventuellement au moins un autre hétéroatome choisi parmi l'azote, l'oxygène et le soufre. Ledit hétérocycle silylé peut être éventuellement substitué par un ou plusieurs groupes hydroxyle ; un ou plusieurs groupes alkyle, un ou plusieurs groupes alkoxy ; un ou plusieurs atomes d'halogène choisis parmi les atomes de fluor, chlore, brome et iode ; un ou plusieurs groupes aryle, avec les groupes alkyle, alkoxy et aryle tels que définis dans le cadre de la présente invention. Parmi les hétérocycles silylés, on peut citer par exemple, le 1-silacyclo-3-pentène ou le 1-méthyl-1,1-dihydrido-2,3,4,5-tétraphényl-1-silacyclopentadiene comme représentés ci-dessous :

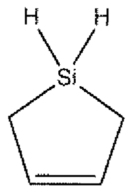

1-silacyclo-3-pentène

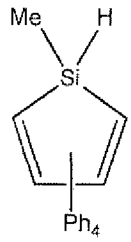

1-méthyl-1-hydrido-2,3,4,5-tétraphényl-1-silacyclopentadiène

Parmi les hétérocycles silylés, on peut encore citer, par exemple, le méthyle siloxane, le 1-phényle-1-silacyclohexane, le 1-sila-bicyclo[2.2.1]héptane, le 1-méthyle-1-silacyclopentane, le 9,9-dihydro-5-silafluorène répondant aux formules semi-développées suivantes :

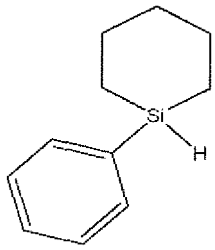

1-phenyl-1-silacyclohexane

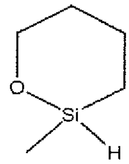

methyl siloxane

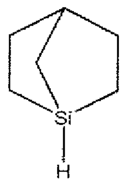

1-sila-bicyclo[2.2.1]heptane

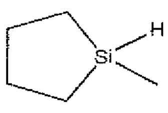

1-methyl-1-silacyclopentane

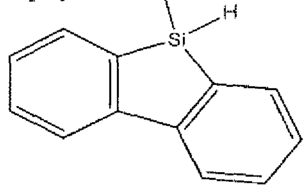

9,9-dihydro-5-silafluorene

Par polyol on entend un composé organique caractérisé par la présence d'un certain nombre de groupements hydroxyle (-OH). Dans le cadre de cette invention un composé polyol contient au moins deux groupements hydroxyle. Plus précisément, on entend par polyol un composé de formule $Y\text{-}(OH)_m$, dans lequel m est supérieur ou égale à 1, et Y est choisi parmi un ou plusieurs groupes alkyle, un ou plusieurs groupes alkoxy, un ou plusieurs groupes siloxy, un ou plusieurs groupes aryle, un ou plusieurs groupes hétéroaryle avec les groupes alkyle, alkoxy, siloxy, aryle et hétéroaryle, tels que définis dans le cadre de la présente invention.

Par groupe « amino », on entend un groupe de formule $-NR^4R^5$, dans laquelle :

- $R^4$ et $R^5$ représentent, indépendamment l'un de l'autre, un atome d'hydrogène, un groupe alkyle, un groupe alcényle, un groupe alcynyle, un groupe aryle, un groupe hétéroaryle, un hétérocycle, un groupe silylé, un groupe siloxy, avec les groupes alkyle, alcényle, alcynyle, aryle, hétéroaryle, hétérocycle, silylé, et siloxy, tels que définis dans le cadre de la présente invention ; ou
- $R^4$ et $R^5$, pris ensemble avec l'atome d'azote auquel ils sont liés, forment un hétérocycle éventuellement substitué par un ou plusieurs groupes hydroxyle ; un ou plusieurs groupes alkyle ; un ou plusieurs groupes alkoxy ; un ou plusieurs atomes d'halogène choisis parmi les atomes de fluor, chlore, brome et iode ; un ou plusieurs groupes nitro ($-NO_2$) ; un ou plusieurs groupes nitrile (-CN) ; un ou plusieurs groupes aryle ; avec les groupes alkyle, alkoxy et aryle tels que définis dans le cadre de la présente invention.

Dans le cadre de l'invention, la subérine désigne un polymère biosourcé principalement trouvé dans les plantes supérieures. Cette substance organique cireuse est imperméable et se trouve sur les parois cellulosiques de certaines cellules végétales, en particulier celles du liège dont elle constitue le principal constituant. La subérine contient deux domaines : celui des polyaliphatiques et celui des polyaromatiques essentiellement formé de dérivés d'acide hydroxycinnamique. La composition exacte de la subérine varie en fonction de l'espèce. Une représentation de la structure de la subérine est montrée en Figure 3.

Le tannin désigne un polymère bio-sourcé contenu dans de nombreux végétaux. Cette substance organique peut être par exemple contenue dans les feuilles (sumac) les écorces et les bois (ex : chêne, acacia) et/ou les racines (badan) des végétaux. Ce composé polyphénolique amorphe est surtout utilisé dans le tannage des peaux pour faire du cuir, la fabrication des encres ou en pharmacologie. Il existe trois grandes catégories de tannins : les tannins hydrolysables, les tannins non hydrolysables ou condensés, et les phlorotannins. Cette définition englobe les pseudo-tannins qui sont des tannins de faible poids moléculaire liés à d'autres composés.

Par tannins hydrolysables, au sens de l'invention, on entend des composés constitués des mélanges de polygalloyles glucoses (ce sont des polymères de molécules formées à partir de dérivés de l'acide gallique et le β-D-glucose comme par exemple dans le cas de l'acide tannique) et/ou de dérivés poly-galloyles de l'acide quinique contenant entre 3 et 12 unités d'acide gallique par molécule. Ces composés contiennent essentiellement des liaisons esters liant les unités aromatiques à un polyol, ce qui facilite son hydrolyse par des acides ou des bases faibles.

Par gallotannins, on entend des tannins hydrolysables dérivés de l'acide gallique, dans lesquels l'acide gallique est lié par des liaisons ester à un polyol central. Dans ces composés, les unités galloyles peuvent de même subir des couplages croisés oxydants (en anglais oxidative cross coupling) ou bien des réactions d'estérification. Il existe plusieurs types de gallotannins répartis essentiellement suivant leur composition chimique comme par exemple : les galloyles glucose (ce sont des molécules formées à partir d'une liaison entre l'acide gallique et le β-D-glucose), les galloyles des acides quiniques, les galloyles des acides shikimiques. La Figure 4 est une représentation de la structure de l'acide tannique aussi appelé β-1,2,2,3,6-Pentagalloyl-O-D-Glucose et appartenant à la classe des gallotannins.

Par ellagitannin, au sens de l'invention, on entend des gallotannins ou des groupements galloyles ayant subi un couplage oxydant C-C. Ce couplage intramoléculaire se réalise pour la plupart des plantes entre les atomes de carbones : C2 et C3 ou bien entre C4 et C6. Ce type de polyphénol forme généralement des macrocycles, alors que ceci n'est pas observable avec les gallotannins.

Par catalyseur, au sens de l'invention, on entend tout composé capable de modifier, notamment en augmentant, la vitesse de la réaction chimique à laquelle il participe, et qui est régénéré à la fin de la réaction. Cette définition englobe à la fois les catalyseurs, c'est-à-dire les composés qui exercent leur activité catalytique sans avoir besoin de subir une quelconque modification ou conversion, et les composés (appelés également pré-catalyseurs) qui sont introduits dans le milieu réactionnel et qui y sont convertis en un catalyseur. Dans le procédé de l'invention, le catalyseur est une molécule organique ou organométallique, c'est-à-dire qu'elle peut contenir ou pas des ions métalliques ou des éléments de transition. Toutefois, les catalyseurs organiques présentent l'avantage de permettre de s'affranchir des problèmes de toxicité généralement observés pour les catalyseurs métalliques ainsi que des problèmes de coûts associés à l'utilisation de métaux précieux.

Il est en particulier nécessaire que le catalyseur soit choisi en tenant compte notamment de son encombrement stérique, de son aptitude à activer le composé silane et de sa solubilité dans le milieu réactionnel.

Le catalyseur peut être un catalyseur organique choisi parmi :

- les carbocations de formule $(X^1)_3C^+$ avec $X^1$ représentant un atome d'hydrogène, un groupe alkyle, un groupe aryle, un groupe alkoxy, un groupe silyle, un groupe siloxy et un atome d'halogène, tels que définis ci-dessus, lesdits carbocations étant choisis parmi le cation trityle ($(C_6H_5)_3C^+$), le tropilium $(C_7H_7)^+$, le cation benzylique ($C_6H_5CH_2^+$), le cation allylique ($CH_3$-$CH^+$-$CH=CH_2$), le méthylium ($CH_3^+$), le cyclopropylium ($C_3H_5^+$), le carbocation cyclopropylique de formule $C_3H_5$-$C^+R^1R^2$ avec $R^1$ et $R^2$ comme définis ci-dessus, ledit carbocation cyclopropylique étant choisi parmi le carbocation diméthyle cyclopropylique et le carbocation dicyclopropylique, le cation triazabicyclodécène (TBD), l'acylium $(R^1$-$C=O)^+$ avec $R^1$ comme défini ci-dessus et choisi parmi le méthyle, le propyle et le benzyle, le benzénium $(C_6H_5)^+$, le cation norbornyle $(C_7H_{11})^+$ ;
- les oxoniums choisis parmi $(CH_3)_3O^+BF_4^-$ (réactif de Meerwein) et $(CH_3CH_2)_3O^+BF_4^-$ ;
- un ion silylium choisi parmi $Et_3Si^+$ et $Me_3Si^+$ ;
- les cations disilyles ayant un hydrure pontant choisis parmi les formules indiquées ci-dessous

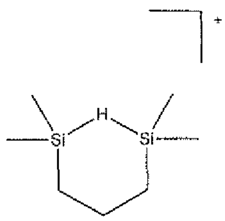 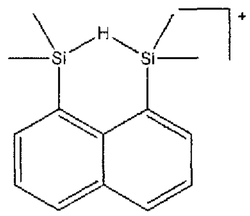 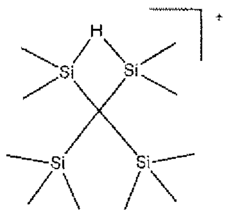

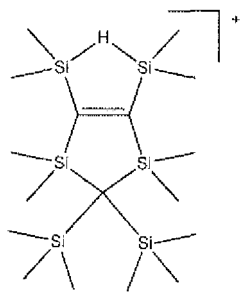

Les carbocations cités ci-dessus sont commerciaux ou peuvent être facilement synthétisés par l'homme du métier par différents procédés de synthèse, par exemple : le procédé de bassin de cation (cation pool), le procédé redox interne, le procédé utilisant un groupement partant, les procédés utilisant des acides de Lewis ou de Bronsted. Ces procédés sont décrits dans les références suivantes : R. R. Naredla et D. A. Klumpp, Chem. Rev. 2013, 113, pages 6905-6948 ; M. Saunders. et H. A. Jimenez-Vazquez, Chem. Rev. 1991, 91, pages 375-397.

Il est à noter que le contre ion anionique de l'ion silylium, des carbocations et des cations disilyles précités est un halogénure choisi parmi $F^-$, $Cl^-$, $Br^-$ et $I^-$, ou un anion choisi parmi $BF_4^-$, $SbF_6^-$, $B(C_6F_5)_4^-$, $B(C_6H_5)_4^-$, $TfO^-$ ou $CF_3SO_3^-$, $PF_6^-$.

Le catalyseur organique peut être de préférence le triphénylcarbénium tetrakis(perfluorophenyl)borate $[(Ph)_3C^+B(C_6F_5)_4]^-$.

Dans le procédé de l'invention, le catalyseur peut être également organométallique. Selon la présente invention un catalyseur organométallique peut être choisi parmi les complexes d'iridium répondant à la formule (II) représentés ci-dessous, dans laquelle

- $R^6$ représente un groupe alkyle ou aryle tel que défini précédemment, et de préférence un groupe tert-butyle ;
- $R^7$ représente un atome d'hydrogène ou un groupe alkyle tel que défini précédemment, et de préférence un atome d'hydrogène ;
- $X^2$ représente un groupe $-CH_2-$ ou un atome d'oxygène, et de préférence un atome d'oxygène ;
- $Y^-$ représente un contre ion choisi parmi $B(C_6F_5)_4^-$, et $B(C_6H_5)_4$, et de préférence $B(C_6F_5)_4^-$ ; et
- S représente une molécule de solvant, coordonnée au complexe, choisi parmi le diméthylesulfoxyde (DMSO), l'acétonitrile ($CH_3CN$) et l'acétone ($CH_3COCH_3$), et de préférence l'acétone.

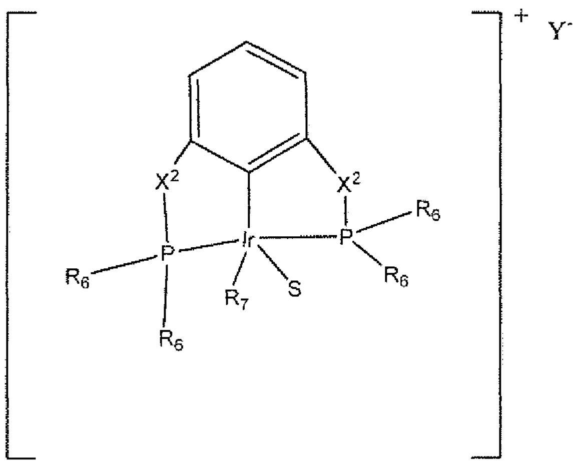

(II)

De préférence, le catalyseur d'iridium est $[(POCOP)Ir(H)(acétone)]^+B(C_6F_5)_4^-$ avec (POCOP) représentant le 2,6-bis(di-tert-butylphosphinito)phényle. Ce catalyseur peut être préparé selon les procédés décrits par I. Gottker-Schnetmann, P. White, et M. Brookhart, J. Am. Chem. Soc. 2004, 126, pages 1804-1811 ; et par J. Yang et M. Brookhart, J. Am. Chem. Soc. 2007, 129, pages 12656-12657.

Selon la présente invention un catalyseur organométallique peut être aussi choisi parmi les complexes de ruthénium répondant à la formule (III) ci-dessous :

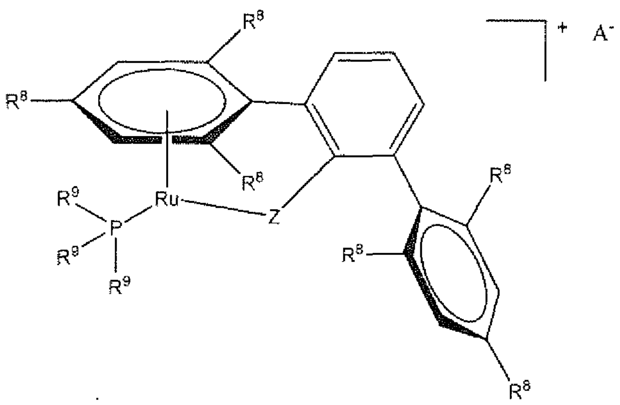

(III)

dans laquelle

- $R^8$ représente un atome d'hydrogène ou un groupe alkyle tel que défini précédemment, $R^8$ étant de préférence un groupe méthyle ;
- $R^9$ représente un aryle ou un groupe alkyle tel que défini précédemment, lesdits groupes aryle et alkyle étant éventuellement substitués, $R^9$ étant de préférence $p$-$FC_6H_4$ ;
- Z représente un groupe $-CH_2-$, un atome d'oxygène ou un atome de soufre, Z étant de préférence un atome de soufre ; et
- $A^-$ représente un contre ion choisi parmi $B(C_6F_5)_4^-$ et $[CHB_{11}H_5Cl_6]^-$, $A^-$ étant de préférence $B(C_6F_5)_4^-$.

Ce type de catalyseur peut être préparé selon les procédés décrits par T. Stahl , H. F. T. Klare, and M. Oestreich, J. Am. Chem. Soc., 2013, 135, pages 1248-1251.

Le catalyseur peut également être de type acide de Lewis choisi parmi :

- les composés de bore de formule $B(X^3)_3$ avec $X^3$ représentant un atome d'hydrogène, un groupe alkyle, un groupe aryle, un groupe alkoxy tels que définis précédemment, lesdits composés de bore étant choisis parmi $BF_3$, $BF_3(Et_2O)$, $BCl_3$, $BBr_3$, le triphényle hydroborane, le tricyclohexyle hydroborane, $B(C_6F_5)_3$, le B-méthoxy-9-borabicyclo[3.3.1]nonane (B-méthoxy-9-BBN), le B-benzyl-9-borabicyclo[3.3.1]nonane (B-benzyl-9-BBN) ;
- les composés boréniums Me-TBD-$BBN^+$, les dérivés borenium ferrocène répondant à la formule

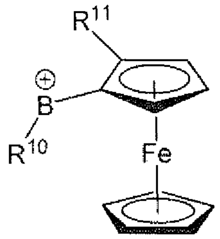

borenium ferrocène

dans laquelle $R^{10}$ et $R^{11}$ sont tels que définis précédemment, par exemple, $R^{10}$= phényle et $R^{11}$= 3,5-diméthylpyridyle (J. Chen, R. A. Lalancettea et F. Jäkle, Chem. Commun., 2013,49, pages 4893-4895) ;
- les composés de l'aluminium choisis parmi $AlCl_3$, $AlBr_3$, l'isopropoxyde d'aluminium $Al(O\text{-}i\text{-}Pr)_3$, l'éthanoate d'aluminium $(Al(C_2H_3O_2))$, le sel de Krossing $[Ag(CH_2Cl_2)]\{Al[OC(CF_3)_3]_4\}$, le $Li\{Al[OC(CF_3)_3]_4\}$, les composés cationiques d'aluminium de formule $(X^4)_2Al^+$ avec $X^4$ étant un atome d'halogène, un groupe alkoxy, un groupe alkyle tels que définis précédemment comme, par exemple, $Et_2Al^+$ ;
- les composés d'indium choisis parmi $InCl_3$, $In(OTf)_3$ ;
- les composés de fer choisis parmi $FeCl_3$, $Fe(OTf)_3$ ;
- les composés de l'étain choisis parmi $SnCl_4$, $Sn(OTf)_2$ ;
- les composés du phosphore choisis parmi $PCl_3$, $PCl_3$, $POCl_3$ ;
- les composés trifluorométhanesulfonates ou triflates $(CF_3SO_3^-)$ de métaux de transitions et des lanthanides choisis

parmi le triflate de scandium, le triflate de ytterbium, le triflate d'yttrium, le triflate de cérium, le triflate de samarium, le triflate de niodinium.

Dans le cadre de la présente invention $OTf^-$ représente l'ion triflate ou trifluorométhanesulfonate de formule $CF_3SO_3^-$: les termes triflate ou trifluorométhanesulfonate, $OTf^-$ ou $CF_3SO_3^-$ peuvent donc être utilisés indifféremment pour désigner la même entité.

La préparation des dérivés borenium ferrocène est décrite par J. Chen, R. A. Lalancettea et F. Jäkle, Chem. Commun., 2013,49, pages 4893-4895 ; la préparation des sels de Krossing est décrite par I. Krossing, Chem.-Eur. J., 2001, 7, page 490 ; et la préparation de $Et_2Al^+$ est décrite par M. Khandelwal et R. J. Wehmschulte, Angew. Chem. Int. Ed. 2012, 51, pages 7323 -7326.

Le catalyseur de type acide de Lewis est de préférence choisi parmi $BF_3$ ; $InCl_3$ ; le dérivé borenium ferrocène tels que définis précédemment dans lequel $R^{10}$= phényle et $R^{11}$= 3,5-diméthylpyridyle.

Les catalyseurs peuvent, le cas échéant, être immobilisés sur des supports hétérogènes afin d'assurer une séparation facile dudit catalyseur et/ou son recyclage. Lesdits supports hétérogènes peuvent être choisis parmi les supports à base de gel de silice et de polymères plastiques comme, par exemple, le polystyrène ; les supports carbonés choisis parmi les nanotubes de carbone ; le carbure de silice ; l'alumine ; et le chlorure de magnésium ($MgCl_2$).

Certaines des abréviations utilisées dans le cadre de l'invention sont représentées ci-dessous :

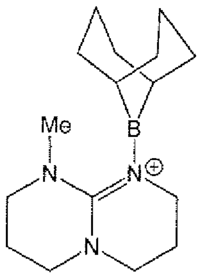
Me-TBD-BBN⁺

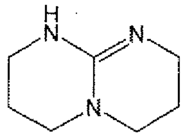
TBD

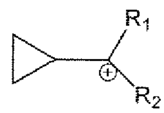
carbocation cyclopropyl

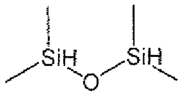
TMDS

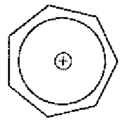
cation tropylium

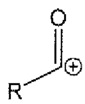
cation acylium

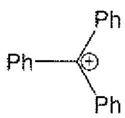
cation trityl

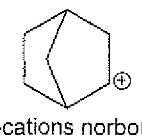
carbocations norbornyl

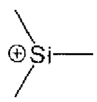
cation silylium

Selon un mode de réalisation particulier de l'invention, le procédé de dépolymérisation, met en oeuvre un composé silane de formule (I) dans laquelle $R^1$, $R^2$ et $R^3$ représentent, indépendamment l'un de l'autre, un atome d'hydrogène ; un groupe hydroxyle ; un groupe alkyle choisi parmi le méthyle, l'éthyle, le propyle, le butyle, et leurs isomères ramifiés ; un groupe alkoxy dont le radical alkyle est choisi parmi le méthyle, l'éthyle, le propyle, le butyle et leurs isomères ramifiés ; un groupe aryle choisi parmi le phényle et le benzyle ; un groupe aryloxy dont le radical aryle est choisi parmi le phényle et le benzyle ; un groupe siloxy ($-O-Si(X)_3$) dont chaque X, indépendamment l'un de l'autre, est choisi parmi un atome d'hydrogène, un groupe alkyle choisi parmi le méthyle, l'éthyle, le propyle, un groupe aryle choisi parmi le phényle et le benzyle, un organosilane polymérique de formule générale

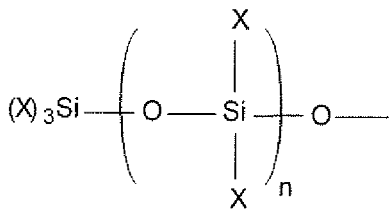

dans laquelle X est tel que défini ci-dessus et n est un nombre entier compris entre 1 et 20000, avantageusement entre 1 et 5000, plus avantageusement entre 1 et 1000 ; lesdits groupes alkyle, alkoxy, aryle, aryloxy, siloxy et aryle étant éventuellement substitués.

Selon un autre mode de réalisation particulier de l'invention, le procédé de dépolymérisation, met en oeuvre un composé silane de formule (I) dans laquelle $R^1$, $R^2$ et $R^3$ représentent, indépendamment l'un de l'autre, un groupe hydroxyle ; un groupe alkyle choisi parmi le méthyle, l'éthyle, le propyle, le butyle, et leurs isomères ramifiés ; un groupe alkoxy dont le radical alkyle est choisi parmi le méthyle, l'éthyle, le propyle, le butyle et leurs isomères ramifiés ; un groupe aryle choisi parmi le phényle et le benzyle ; un groupe aryloxy dont le radical aryle est choisi parmi le phényle et le benzyle ; un groupe siloxy ($-O-Si(X)_3$) dont chaque X, indépendamment l'un de l'autre, est choisi parmi un atome d'hydrogène, un groupe alkyle choisi parmi le méthyle, l'éthyle, le propyle, un groupe aryle choisi parmi le phényle et le benzyle, un organosilane polymérique de formule générale

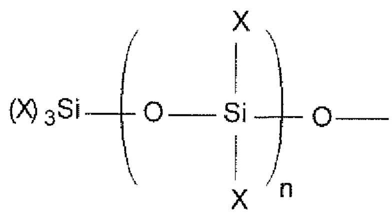

dans laquelle X est tel que défini ci-dessus et n est un nombre entier compris entre 1 et 20000, avantageusement entre 1 et 5000, plus avantageusement entre 1 et 1000; lesdits groupes alkyle, alkoxy, aryle, aryloxy, siloxy et aryle étant éventuellement substitués.

Dans un mode de réalisation particulier de l'invention, le procédé de dépolymérisation, met en oeuvre un composé silane de formule (I) dans laquelle $R^1$, $R^2$ et $R^3$ représentent, indépendamment l'un de l'autre, un atome d'hydrogène ; un groupe alkyle choisi parmi le méthyle, l'éthyle, le propyle et son isomère ramifié ; un groupe aryle choisi parmi le benzyle et le phényle ; un groupe siloxy choisi parmi le polydiméthylsiloxane (PDMS), le polyméthylhydroxysiloxane (PMHS) et le tétraméthyldisiloxane (TMDS).

Dans un autre mode de réalisation particulier de l'invention, le procédé de dépolymérisation, met en oeuvre un composé silane de formule (I) dans laquelle $R^1$, $R^2$ et $R^3$ représentent, indépendamment l'un de l'autre, un groupe alkyle choisi parmi le méthyle, l'éthyle, le propyle et son isomère ramifié ; un groupe aryle choisi parmi le benzyle et le phényle ; un groupe siloxy choisi parmi le polydiméthylsiloxane (PDMS), le polyméthylhydroxysiloxane (PMHS) et le tétraméthyldisiloxane (TMDS).

Le PMHS et le TMDS qui sont deux sous-produits de l'industrie de la silicone, peuvent être valorisés dans le procédé de dépolymérisation selon l'invention. L'utilisation de deux sous-produits industriels pour créer des molécules ayant une haute valeur ajoutée, est économiquement très avantageuse.

Comme déjà indiqué, le procédé de dépolymérisation se réalise dans des conditions opératoires très douces : température et pression faibles, durée de réaction relativement courte. Une conversion totale du ou des réactifs de départ peut être obtenue en quelques minutes à quelques heures. Il est à noter que la conversion est exprimée par rapport au matériau polymère oxygéné.

Dans le procédé selon l'invention, la dépolymérisation peut être effectuée sous une pression d'un ou d'un mélange de gaz inerte(s) choisi(s) parmi l'azote et l'argon, ou des gaz générés par le procédé notamment du méthane et d'hydrogène. La pression peut être comprise entre 0,2 et 50 bars, de préférence entre 0,2 et 30 bars, plus préférentiellement entre 1 et 20 bars, bornes incluses.

La dépolymérisation peut être réalisée à une température comprise entre 0 et 150°C, de préférence entre 0 et 125°C, plus préférentiellement entre 25 et 70°C, bornes incluses.

La durée de la réaction dépend du taux de conversion du composé silane de formule (I), de la nature du matériau polymère de départ ainsi que du taux de silylation souhaité dans les composés finaux visés.

La dépolymérisation peut être effectuée pendant une durée de 1 minute à 200 heures, avantageusement de 1 minute à 48 heures, de préférence de 10 minutes à 48 heures, bornes incluses.

La dépolymérisation, en particulier la réaction entre les différents réactifs, peut avoir lieu dans un ou un mélange d'au moins deux solvant(s) choisi(s) parmi :

- les éthers silylés, de préférence, choisis parmi le 1,1,1,3,3,3-hexaméthyldisiloxane ($(Me_3Si)_2O$), le 1,1,1,3,3,3-hexaéthyldisiloxane ($(Et_3Si)_2O$).
- les hydrocarbures, de préférence, choisis parmi le benzène, le toluène, le pentane et l'hexane ;
- les sulfoxydes, de préférence, choisis parmi le diméthylesulfoxyde (DMSO) ;
- les halogénures d'alkyle, de préférence, choisis parmi le chloroforme, le chlorure de méthylène, le chlorobenzène, le dichlorobenzène.

Les silanes de formule (I) et les catalyseurs utilisés dans l'étape de dépolymérisation sont, en général, des composés commerciaux ou peuvent être préparés par les procédés connus de l'homme du métier.

Le rapport molaire entre le composé silane de formule (I) et le matériau polymère de départ dépend du type du matériau polymère de départ employé (type du matériau polymère et la quantité d'additifs et de contaminants présents dans ledit matériau polymère) et du type de composés finaux souhaités (obtention de fonctions siloxy ($-O-SiR^1R^2R^3$) dans le produit final ou réduction totale jusqu'au dérivé désoxygéné). En effet, comme le procédé de dépolymérisation de l'invention clive les liaisons carbone sp3-oxygène, les liaisons silylées (-C-O-Si-) peuvent être facilement réduites en liaison -C-H. La sélectivité par rapport au type de composés finaux dépendra du nombre d'équivalents de composé silane de formule (I) ajouté.

Ainsi, dans le cadre de la présente invention, le rapport molaire entre le composé silane de formule (I) et le matériau polymère oxygéné peut être compris entre 0,1 et 20, de préférence entre 0,5 et 10, bornes incluses.

La quantité de catalyseur utilisé dans le procédé de dépolymérisation peut être de 0,001 à 1 équivalent molaire, de préférence de 0,001 à 0,9 équivalent en masse, plus préférentiellement de 0,01 à 0,7 équivalent molaire, encore plus préférentiellement de 0,01 à 0,5 équivalent molaire, bornes incluses, par rapport au nombre de mole initial du matériau polymère oxygéné de départ.

Après la dépolymérisation, les composés résultants peuvent être sous forme silylée. Une simple hydrolyse dans des conditions bien connues de l'homme du métier peut alors conduire aux composés aromatiques correspondant sous leurs formes non silylées.

Dans le cadre de la présente invention, par hydrolyse on entend un procédé de transformation des groupements siloxy présents dans des composés silylés issus de dépolymérisation de la lignine, en groupements hydroxyles, par une réaction de désilylation. Cette transformation peut se réaliser dans des conditions acides ou basiques ou bien en présence d'ions fluorures, ces conditions étant bien connues de l'homme du métier. Dans le cadre de la présente invention, le procédé d'hydrolyse est, de préférence, choisi parmi : HCl ou $H_2SO_4$ 2 M dans le THF ; NaOH ou KOH 10 % dans un mélange eau/THF ; $TBAF.3H_2O$ dans le THF ; solution commerciale de TBAF (1M) dans le THF.

Une simple filtration peut permettre de récupérer le catalyseur éventuellement supporté et d'éliminer les éventuels sous-produits.

Le procédé de l'invention permet la préparation de composés comportant une ou plusieurs fonctions siloxy ($-OSiR^1R^2R^3$) ou de composés dépourvus d'atome d'oxygène. Dans le cas où le matériau polymère oxygéné de départ comporte plusieurs fonctions clivables, la dépolymérisation conduira alors à un mono- ou poly-siloxane qui après l'hydrolyse conduira à l'obtention d'un alcool simple ou d'un polyol. Les composés issus de la dépolymérisation sont obtenus avec une bonne pureté, c'est-à-dire, une pureté supérieure ou égale à 90 % molaire, de préférence comprise entre 90 et 99,9 % molaire. La pureté molaire peut être déterminée par une analyse spectroscopique ou chromatographique par exemple la RMN du proton (RMN $^1H$) ou la chromatographie en phase gazeuse couplée à la spectrométrie de masse (GC-MS). En effet, dans le procédé de l'invention, les composés formés peuvent être facilement purifiés par les techniques de séparation bien connues de l'homme du métier et classiquement utilisées dans ce domaine, comme par exemple, la chromatographie sur colonne, la distillation pour les produits volatiles, etc. Les composés obtenus étant en général des petites molécules c'est-à-dire des molécules ayant une masse molaire inférieure à 600 g/mol, leur séparation des produits secondaires éventuellement formés qui sont en général des oligomères avec des liaisons ne pouvant être clivées par le procédé de l'invention, est facile compte tenu des propriétés physico-chimiques très différentes desdits oligomères et des composés obtenus.

Le procédé de l'invention est d'une grande robustesse car il est résistant aux contaminants éventuellement présents dans les matériaux polymères commerciaux (comme les traces d'eaux et les traces de métaux) ainsi qu'aux additifs comme les colorants, ajoutés aux matériaux polymères comme par exemple les plastiques.

Le procédé de l'invention peut être utilisé pour le recyclage de matériaux composites comme les résines contenant du PVC et du PET et qui sont problématiques à recycler. En effet, les liaisons esters du PET sont clivées alors que les liaisons C-C du PVC restent intactes.

Ce procédé peut apporter une solution au stockage des déchets en permettant le recyclage du mélange de déchets de PET et de PLA. En effet, étant donné leur ressemblance au niveau des propriétés physiques et visuelles

les plastiques (PET) et les bioplastiques (PLA) sont couramment mélangés. Cependant, leur séparation est très coûteuse et les procédés de recyclage actuels ne permettent pas de recycler les deux polymères en même temps. Il existe donc un vrai problème de recyclage de mélanges de plastiques (PET) et de bio-plastiques (PLA). Le procédé de l'invention permet de recycler un mélange de PET et de PLA soit en clivant le PET sélectivement soit en coupant le PET et le PLA et ce en fonction des conditions opératoires choisies.

Ainsi, l'invention a pour objet l'utilisation du procédé de dépolymérisation de l'invention pour le recyclage de matériaux plastiques contenant au moins un polymère oxygéné au sens de l'invention. En particulier, l'invention a pour objet l'utilisation du procédé de l'invention pour le recyclage des plastiques ou des mélanges de plastiques contenant au moins un polymère oxygéné, c'est-à-dire un polymère ou copolymère dont la chaîne principale comporte des fonctions esters et/ou carbonates, comme par exemple le PLA, le PET, le PC-BPA.

L'invention a encore pour objet un procédé de recyclage de matériaux plastiques ou des mélanges de matériaux plastiques contenant au moins un polymère oxygéné au sens de l'invention, c'est-à-dire un polymère ou copolymère dont la chaîne principale comporte des fonctions esters et/ou carbonates, comme par exemple le PLA, le PET, le PC-BPA, ledit procédé comprenant une étape de dépolymérisation de matériaux polymères oxygénés selon l'invention.

Dans le procédé de recyclage, l'étape de dépolymérisation de matériaux polymères oxygénés selon l'invention peut être précédée d'une étape de préparation ou de transformation desdits matériaux plastiques. A titre d'exemple, une étape de broyage, d'extrusion, de pulvérisation ou de micronisation peut être utilisée afin d'augmenter la surface de matériaux et améliorer les performances de l'étape de dépolymérisation selon l'invention.

A l'issu du procédé de dépolymérisation de l'invention et après hydrolyse, des composés aromatiques de poids moléculaire inférieure à 600 g/mol, comme par exemple, le benzène, le toluène, les xylènes (BTX), les coniférols substitués, le phénol, les polyols aromatiques, les quinines, les dérivés de catéchols et d'hydroxycatéchol peuvent être obtenus lorsque le matériau polymère oxygéné de départ contient des motifs aromatiques. Ces composés peuvent être utilisés comme carburant, intermédiaires de synthèse, matières premières dans les secteurs de la construction, dans l'industrie pétrochimique, dans l'industrie électrique, dans l'industrie électronique, dans l'industrie de textile, dans l'industrie aéronautique, dans l'industrie pharmaceutique, dans l'industrie cosmétique, dans l'industrie agrochimique.

Eventuellement, le procédé de recyclage de matériaux plastiques ou de mélanges de matériaux plastiques peut être suivi d'une étape de transformation chimique ou de mise en forme physique des produits de dépolymérisation fournis par le présent procédé. A titre d'exemple, les produits silylés obtenus par dépolymérisation de matériaux polymères oxygénés peuvent être hydrolysés en présence d'anions fluorure pour fournir des alcools.

L'invention a donc pour objet un procédé de préparation des composés aromatiques mono-, di-, et/ou tri-cycliques dont chaque cycle peut éventuellement être mono-, di- et /ou tri-oxygénés comprenant une étape de dépolymérisation de matériaux polymères oxygénés selon le procédé de l'invention. Dans ce procédé, après l'étape de dépolymérisation de matériaux polymères oxygénés selon l'invention, une étape d'hydrolyse peut éventuellement s'avérer nécessaire.

Eventuellement, le procédé de préparation des composés aromatiques mono-, di-, et/ou tri-cycliques dont chaque cycle peut éventuellement être mono-, di- et /ou tri-oxygénés peut être suivi d'une étape de transformation chimique ou de mise en forme physique des produits de dépolymérisation fournis par le procédé de l'invention. A titre d'exemple, les produits silylés aromatiques obtenus par dépolymérisation de matériaux polymères oxygénés peuvent être hydrolysés en présence d'anions fluorure pour fournir les alcools aromatiques correspondants.

Les composés obtenues par le procédé de dépolymérisation de la présente invention sont de composés aromatiques mono-, di-, et/ou tri-cycliques dont chaque cycle peut éventuellement être mono-, di- et/ou tri-oxygénés qui peuvent être utilisés dans la fabrication de combustibles, de composants électroniques, de polymères plastiques, de caoutchouc, de médicaments, de vitamines, de produits cosmétiques, de parfums, de produits alimentaires, de fils et fibres synthétiques, de cuirs synthétiques, de colles, de pesticides, d'engrais.

D'autres avantages et caractéristiques de la présente invention apparaîtront à la lecture des exemples ci-dessous donnés à titre illustratif et non limitatif et des figures annexées dans lesquelles :

- La Figure 1 représente le procédé de dépolymérisation des matériaux polymères oxygénés selon l'invention. A et B représente deux groupements identiques ou différents choisis parmi un ou plusieurs groupes alkyle éventuellement substitués, un ou plusieurs groupes alcényles éventuellement substitués, un ou plusieurs groupes alcynyle éventuellement substitués, un ou plusieurs groupes aryle éventuellement substitués, un ou plusieurs groupes hétéroaryle éventuellement substitués, avec les groupes alkyle, alcényle, alcynyle, aryle et hétéroaryle tels que définis dans le cadre de la présente invention. n est un nombre entier compris entre 1 et 20000.
- La Figure 2 représente le procédé de dépolymérisation de l'invention pour quelques matériaux polymères oxygénés.
- La Figure 3 représente la structure de la subérine.
- La Figure 4 représente la structure de l'acide tannique aussi appelé β-1,2,2,3,6-Pentagalloyl-O-D-Glucose et appartenant à la classe des gallotannins.

## EXEMPLES SELON LA PRÉSENTE INVENTION

Les réactions de dépolymérisation catalytique de matériaux polymères oxygénés selon l'invention sont présentées dans les Figures 1 et 2.

Dans les exemples ci-après, les polymères oxygénés testés sont le PEG, le PET, le PC-BPA et le PLA). D'autre part, la quantité d'hydrosilane de formule générale (I) nécessaire pour réaliser la dépolymérisation est largement dépendante du type de matériau polymère employé ainsi que le composé recherché. En effet, les composés contenant des fonctions alcools silylés ($C\text{-}O\text{-}SiR_1R_2R_3$) issus de la réaction de dépolymérisation peuvent être désoxygénés par le même procédé pour conduire à des liaisons C-H.

Il est à noter que, par approximation, et afin de calculer le rendement molaire des réactions de dépolymérisation, le matériau de départ est considéré être exclusivement constitué du polymère étudié.

Les rendements obtenus sont de l'ordre de 30 à 99 % molaire par rapport au nombre de mole du polymère oxygéné de départ. Les conversions ont été calculées en se basant sur des analyses spectroscopiques (RMN $^1H$ et RMN $^{13}C$) en utilisant un spectromètre Bruker DPX 200 MHz et par l'ajout d'un étalon interne (le mésitylène ou le diphénylméthane). Les rendements ont été obtenus à l'aide de la chromatographie en phase gazeuse en utilisant comme étalon, le même composé préalablement synthétisé (courbe d'étalonnage externe). Les données de spectrométrie de masse ont été recueillies sur un appareil Shimadzu GCMS-QP2010 Ultra gas chromatograph mass spectrometer équipé avec une colonne capillaire en silice fondue Supelco SLB™-ms (30 m x 0.25 mm x 0.25 μm). Les analyse qualitatives de gaz ont été exécutés à l'aide de la chromatographie en phase gazeuse sur un appareil Shimadzu GC-2010 équipé d'une colonne capillaire Carboxen™ 1006 PLOT (30 mx 0,53 mm).

### Protocole expérimental général de dépolymérisation

1. Sous atmosphère inerte d'argon ou d'azote, l'hydrosilane de formule générale (I), le catalyseur (de 1 à 0,001 équivalents molaire calculée par rapport au nombre de moles de matériau polymère initialement ajouté) et la moitié de la quantité de solvant sont mis sous agitation dans un récipient en verre de volume adapté. La concentration en silane dans le mélange réactionnel varie de 1,0-6,0 $mol.L^{-1}$ (concentration calculée sur la base de la moitié du volume final de solvant introduit).
2. D'autre part, dans un tube de Schlenk, le matériau polymère oxygéné (utilisé comme reçu) est placée sous agitation avec la moitié restante de solvant.
3. La solution contenant le catalyseur et l'hydrosilane est ajoutée lentement (temps d'ajout 5 minutes à 1 heure) à l'aide d'une seringue et sous agitation, au tube de Schlenk. Ce dernier est laissé ouvert afin d'évacuer les gaz produits par la réaction.
4. Après la fin de l'ajout de la solution et l'arrêt du dégagement gazeux, le tube de Schlenk est fermé et est laissé sous agitation. Dans le cas où le matériau de départ est insoluble la solubilisation se réalise pendant le temps de réaction vu que les produits finaux sont solubles dans les solvants utilisés. Le suivi de réaction est réalisé par RMN $^1H$ et par GC-MS.
5. Une fois la réaction terminée (temps de réaction de 1 minute à 24 heures), le solvant ainsi que les composés volatils sont évaporés à l'aide d'une rampe à vide ($10^{-2}$ mbar). L'huile obtenue est purifiée à l'aide d'une chromatographie sur gel de silice en utilisant un gradient d'élution de 100 : 0 % jusqu'à 0 : 100 % de pentane : $CH_2Cl_2$. Il est à noter que les produits liquides qui ont des températures d'ébullition faibles comme le paraxylène, peuvent être purifiés par distillation fractionnée avec un recyclage du solvant.
6. Dans le cas des produits ayant des fonctions siloxy ($-SiOR_1R_2R_3$), ces produits sont hydrolysées en utilisant $TBAF.3H_2O$, pour fournir le produit hydrolysé correspondant. La réaction d'hydrolyse dure de 1 minute à 16 heures. Le produit final est obtenu après purification sur colonne chromatographique en utilisant un gradient d'élution de 100 : 0 % jusqu'à 0 : 100 % de $CH_2Cl_2$ : AcOEt.

Un ensemble de résultats est présenté ci-dessous, donnant des exemples de dépolymérisation de matériaux polymères oxygénés synthétiques et biosourcés.

Les catalyseurs testés sont le $B(C_6F_5)_3$ le $(Ph_3)C^+B(C_6F_5)_3^-$ ainsi que le complexe d'iridium ($[(POCOP)Ir(H)(acétone)]^+B(C_6F_5)_4^-$).

Les hydrosilanes utilisés sont $Et_3SiH$, TMDS et PMHS. Les matériaux polymères oxygénés utilisés sont le PLA, le PEG, le PC-BPA, le PET, la subérine et l'acide tannique. La subérine est obtenue à partir des bouchons de liège utilisé dans les bouteilles de vin commerciales. L'acide tannique utilisé est extrait des extraits des noix de galle de chine. Le PET utilisé est un PET commercial prélevé de bouteilles de Perrier.

**A) Dépolymérisation de polymères oxygénés en présence de $B(C_6F_5)_3$**

**Exemple 1 : Dépolymérisation du PC-BPA avec le triéthylsilane ($Et_3SiH$)**

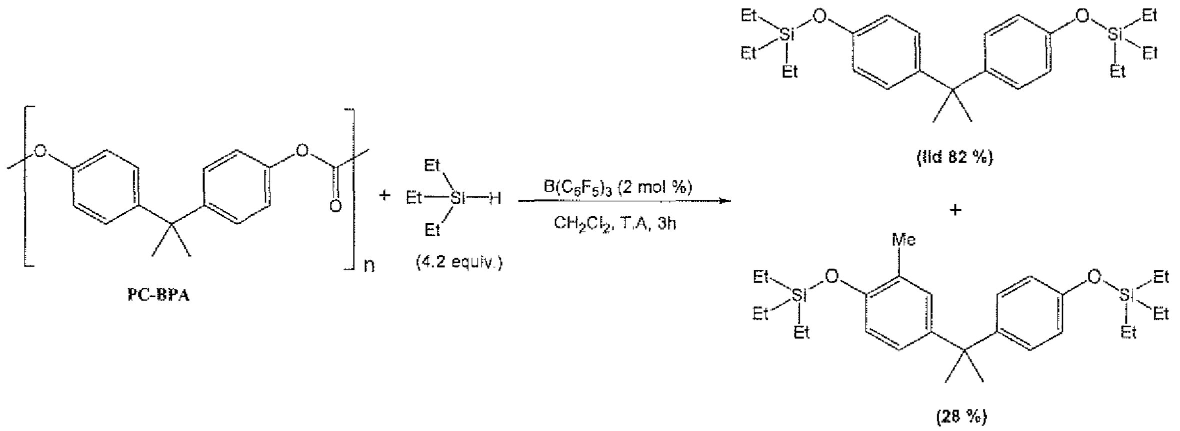


Le PC-BPA commercial (123,2 mg, 0,5 mmol, 1 équiv.) a été ajouté à 1,5 mL de $CH_2Cl_2$. D'autre part, le $B(C_6F_5)_3$ (5,1 mg, 0,01 mmol, 2 mol%) est solubilisé dans un mélange de triéthylsilane (244,2 mg, 2,1 mmol, 4,2 équiv.) et de 1,5 mL de $CH_2Cl_2$. Ensuite, la solution contenant l'hydrosilane et le catalyseur est ajoutée lentement à la solution contenant le polymère préalablement mise sous agitation. Après 3 heures de réaction à température ambiante (20 $\pm$ 5°C), le solvant est évaporé sous vide. Le produit obtenu **IId** est purifié en utilisant les mêmes conditions que celle décrites dans le mode opératoire général. A l'issu de cette purification, le produit **IId** est obtenu avec une grande pureté avec un rendement de 77% par rapport au matériau de départ introduit. Enfin, l'hydrolyse du produit **IId** se réalise en l'agitant à 25°C pendant 2 heures dans une solution de TBAF.$3H_2O$ (2,1 équiv par rapport au nombre de moles de **IId**) dans le THF (3 mL). Le produit hydrolysé (BPA) est obtenu avec un rendement quantitatif, sous forme de solide blanc, après purification sur colonne chromatographique en utilisant les conditions décrites dans le mode opératoire générale.

**Exemple 2: Dépolymérisation du PET en utilisant le triéthylsilane ($Et_3SiH$)**

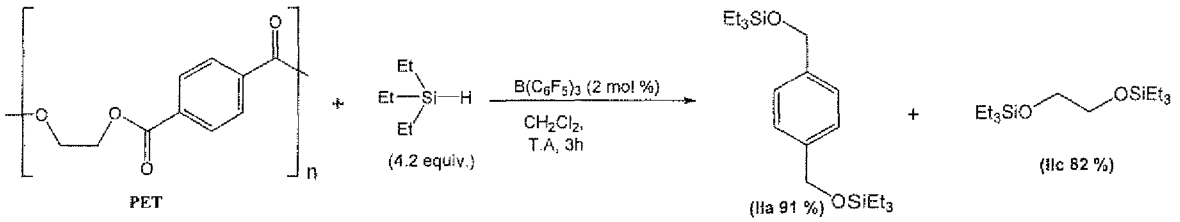


Le même mode opératoire employé pour la dépolymérisation du PC-BPA par $Et_3SiH$ est employé pour la dépolymérisation du PET. Dans ce cas, (96,1 mg, 0,5 mmol, 1 équiv.) de PET sont utilisés avec (244,2 mg, 2,1 mmol, 4,2 équiv.) de triéthylsilane et (5 mg, 0,01 mmol, 2 mol%) de $B(C_6F_5)_3$ Après 3 heures de réaction à température ambiante (20 $\pm$ 5°C) la conversion est totale en **IIa** et **IIc.** La purification des produit se réalise en suivant le même mode opératoire décrit dans l'exemple 1. L'hydrolyse de ces composés dans les conditions de l'exemple 1 conduit à l'obtention de l'éthylène glycol (huile incolore, 72 % de rendement) et du 1,4-phénylènediméthanol (solide blanc, 85% de rendement).

**Exemple 3 : Dépolymérisation du PET avec le polyméthylhydrosiloxane (PMHS)**

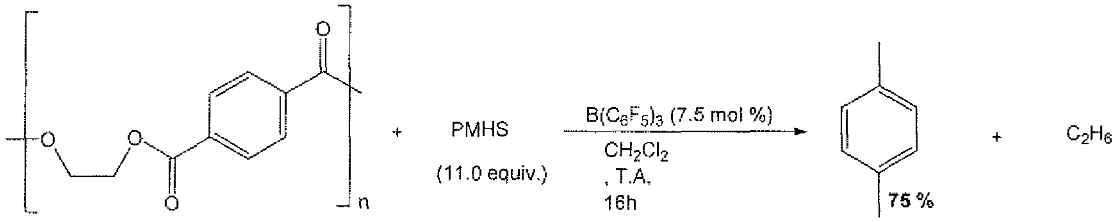


PET

Le même mode opératoire que celui de l'exemple 2 est employé pour la dépolymérisation du PET avec le PMHS. Cependant, les produits obtenus sont l'éthane et le paraxylène. Dans ce cas, (96,1 mg, 0,5 mmol, 1 équiv.) de PET sont utilisés avec (330,7 mg, 5,5 mmol, 11 équiv.) de PMHS, (19,2 mg, 0,04 mmol, 7,5 mol%) de $B(C_6F_5)_3$ et 6 mL de $CH_2Cl_2$. Après 16 heures de réaction, le rendement en paraxylène obtenu est de 75%.

**Exemple 4 : Dépolymérisation du PET avec le tétraméthyldisiloxane (TMDS)**

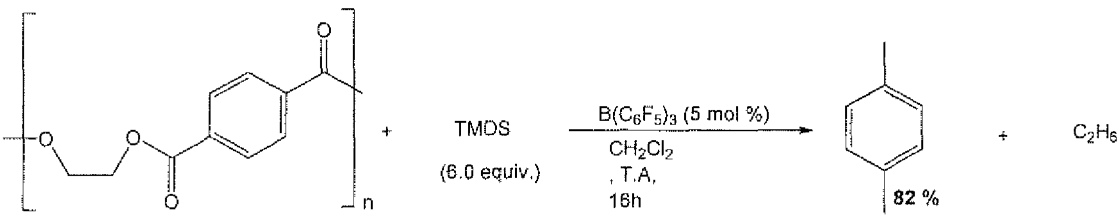


PET

Le même mode opératoire que celui de l'exemple 3 est employé pour la dépolymérisation du PET avec le TMDS. Les produits ont été obtenus sont l'éthane et le paraxylène. (96,1 mg, 0,5 mmol, 1 équiv.) de PET ont été utilisés avec (400,0 mg, 3,0 mmol, 6 équiv.) de TMDS, (12,8 mg, 0,025 mmol, 5 mol%) de $B(C_6F_5)_3$ et 3 mL de $CH_2Cl_2$. Après 16 heures de réaction la conversion est totale et le rendement en paraxylène obtenu est de 82%.

**Exemple 5 : dépolymérisation du PLA en utilisant le triéthylsilane ($Et_3SiH$)**

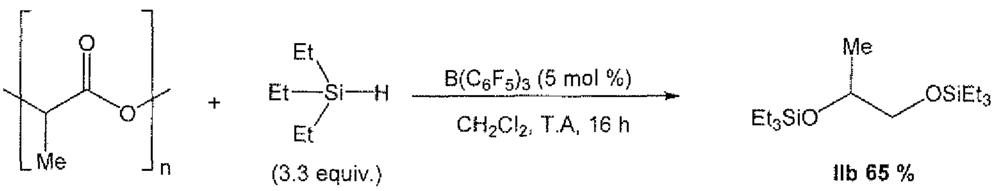


PLA

Le même mode opératoire que celui de l'exemple 2 est employé pour la dépolymérisation du PLA avec le $Et_3SiH$. Le produit **IIb** est obtenu. Dans ce cas, (360,3 mg, 0,5 mmol, 1 équiv.) de PLA sont utilisés avec (191,9 mg, 1,7 mmol, 3,3 équiv.) de $Et_3SiH$, (12,8 mg, 0,025 mmol, 5 mol%) de $B(C_6F_5)_3$ et 3 mL de $CH_2Cl_2$. Après 3 heures de réaction le rendement en produit **IIb** est de 65 %.

**Exemple 6 : Dépolymérisation du PLA avec le polyméthylhydrosiloxane (PMHS)**

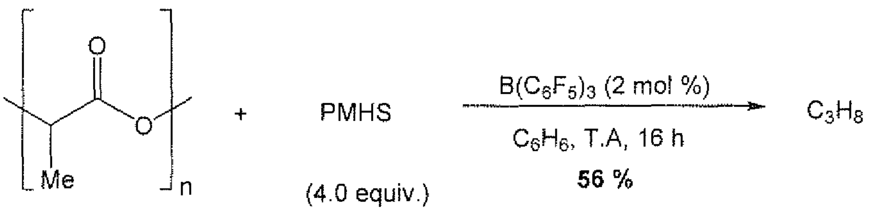


**PLA**

Le même mode opératoire que celui de l'exemple 5 est employé pour la dépolymérisation du PLA avec le PMHS. Le produit obtenu est le propane et le solvant utilisé est le benzène. Dans ce cas, (360,3 mg, 0,5 mmol, 1 équiv.) de PLA sont utilisés avec (120,3 mg, 2,0 mmol, 4 équiv.) de PMHS, (5,1 mg, 0,01 mmol, 2 mol%) de $B(C_6F_5)_3$ et 3 mL de benzène Après 16 heures de réaction, la conversion est de 56 %.

Il est à noter que l'utilisation du $CH_2Cl_2$ comme solvant de réaction conduit à la formation directe d'un gel.

**Exemple 7 : Dépolymérisation du PLA avec le tétraméthyldisiloxane (TMDS)**

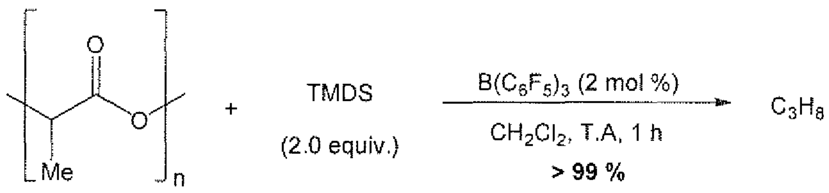


**PLA**

Le même mode opératoire que celui de l'exemple 6 est employé pour la dépolymérisation du PLA avec le TMDS. La réaction a lieu dans le $CH_2Cl_2$. Dans ce cas, (360,3 mg, 0,5 mmol, 1 équiv.) de PLA sont utilisés avec (133,3 mg, 1,0 mmol, 2 équiv.) de TMDS, (5,1 mg, 0,01 mmol, 2 mol%) de $B(C_6F_5)_3$ et 3 mL de $CH_2Cl_2$. Après 1 heure de réaction la conversion est > 99 %.

**Exemple 8 : Dépolymérisation de la gallotannin avec le triéthylsilane ($Et_3SiH$)**

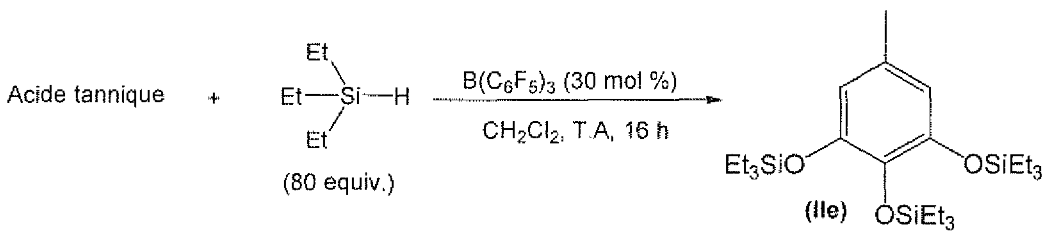


Le même mode opératoire que celui de l'exemple 1 est utilisé pour la dépolymérisation de l'acide tannique avec le triéthylsilane. Dans ce cas, (170,1 mg, 0,1 mmol, 1 équiv.) d'acide tannique ($C_{76}H_{52}O_{46}$) sont utilisés avec (930,2 mg, 8,0 mmol, 80 équiv.) de $Et_3SiH$, (15,4 mg, 0,03 mmol, 30 mol%) de $B(C_6F_5)_3$ et 3 mL de $CH_2Cl_2$ (conditions non optimisées). Après 16 heures de réaction le rendement molaire en produit **IIe** est de 132 % soit 0,13 mmol. La purification du produit est faite en utilisant les mêmes conditions que celle décrites dans le mode opératoire général.

Il est à noter que l'hydrolyse du produit est réalisée dans des conditions quasi similaires à celles décrites dans le mode opératoire général. En effets, cette dernière étape doit se faire sous atmosphère inerte d'argon ou d'azote vu que le produit 5-méthylbenzène-1,2,3-triol s'oxyde directement en quinone en présence d'oxygène.

**Exemple 9 : Dépolymérisation d'un mélange PET+PS avec le triéthylsilane ($Et_3SiH$)**

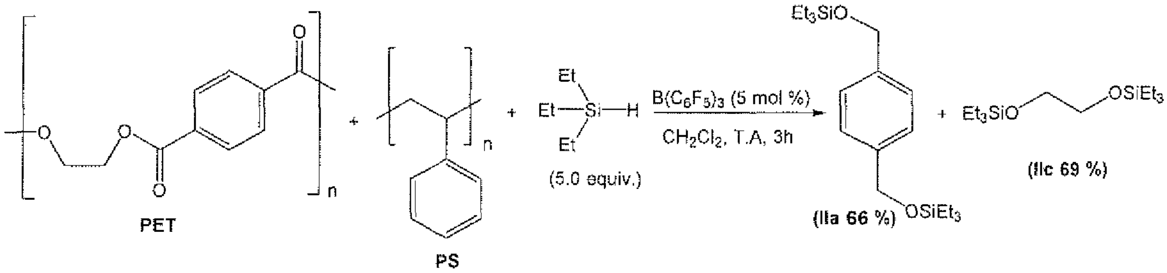


Le même mode opératoire que celui de l'exemple 2 est utilisé pour la dépolymérisation du mélange PET+PS. Dans ce cas, (96,1 mg, 0,5 mmol, 1 équiv.) de PET sont utilisés avec (52,1 mg, 0,5 mmol, 1 equiv.) de PS (polystyrène expansé commercial), (290,7 mg, 2,5 mmol, 5,0 équiv.) de triéthylsilane et (12,8 mg, 0,03 mmol, 5 mol%) de $B(C_6F_5)_3$. Dans 3 mL de $CH_2Cl_2$. Après 3 heures de réaction la conversion est totale en produits **IIa** et **IIc,** le PS n'ayant pas subit une dépolymérisation dans ces conditions. La purification des produits se réalise en suivant le même mode opératoire que celui décrit dans l'exemple 2. L'hydrolyse de ces composés conduit à l'obtention de l'éthylène glycol et du 1,4-phénylènediméthanol.

**Exemple 10: Dépolymérisation d'un mélange PET+PVC+PS avec le triéthylsilane ($Et_3SiH$)**

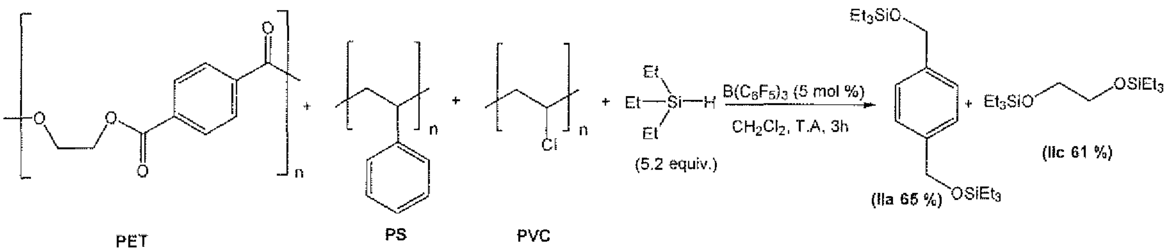


Le même mode opératoire que celui de l'exemple 9 est utilisé pour la dépolymérisation du mélange PET+PS+PVC. Les mêmes quantités de PET et de PS sont utilisées et (31,3 mg, 0,5 mmol, 1 équiv.) de PVC (issu des tuyaux de plomberie) sont employées dans la réaction. Seul le PET est sélectivement dépolymérisé alors que le PVC et le PS ne sont pas dépolymérisés dans ces conditions.

**Exemple 11 : Dépolymérisation sélective du PET dans un mélange PET+PLA avec le triéthylsilane ($Et_3SiH$)**

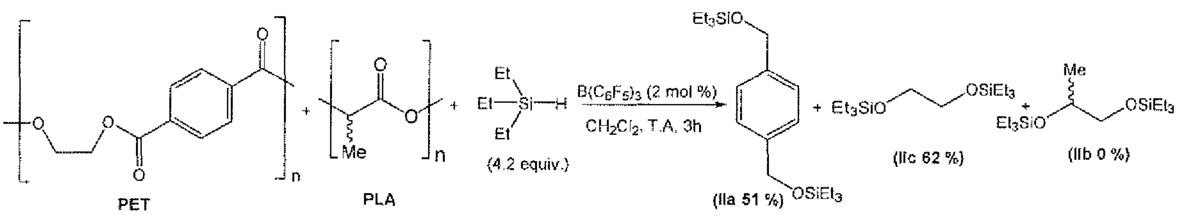


Le même mode opératoire que celui de l'exemple 10 est utilisé pour la dépolymérisation du mélange PET+PLA. Dans ce cas, (96,1 mg, 0,5 mmol, 1 équiv.) de PET sont utilisés avec (360,3 mg, 0,5 mmol, 1 équiv.) de PLA, (244,2 mg, 2,1 mmol, 4,2 équiv.) de triéthylsilane et (5,1 mg, 0,01 mmol, 2 mol%) de $B(C_6F_5)_3$ Dans 3 mL de $CH_2Cl_2$. Après 3 heures de réaction les produits **IIa** et **IIc** sont obtenus. Aucun produit issu de la depolymerisation du PLA n'est observé. La purification des produits se réalise en suivant le même mode opératoire que celui décrit dans l'exemple 2. L'hydrolyse de ces composés conduit à l'obtention de l'éthylène glycol et du 1,4-phénylènediméthanol.

**Exemple 12: Dépolymérisation d'un mélange PET+PLA avec le triéthylsilane ($Et_3SiH$)**

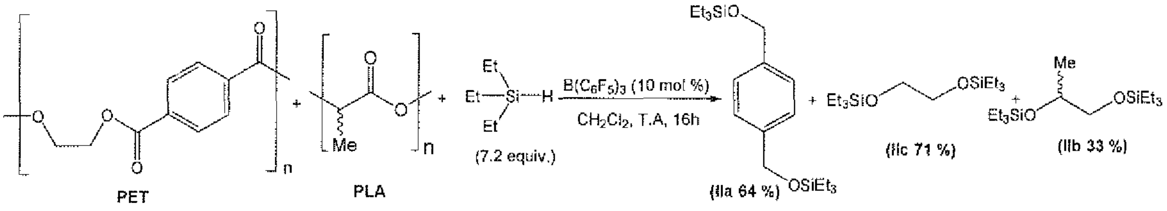


Le même mode opératoire que celui de l'exemple 11 est utilisé pour la dépolymérisation du mélange PET+PLA. Toutefois, la quantité d'hydrosilane utilisée est de (418,6 mg, 3,6 mmol, 7,2 équiv.) et la quantité de catalyseur utilisée est de (25,6 mg, 0,06 mmol, 10 mol%). Après 16 heures de réaction les produits **IIa, IIb** et **IIc** sont obtenus. L'hydrolyse de ces composés conduit à l'obtention de l'éthylène glycol, du propane-1,2-diol et du 1,4-phénylènediméthanol.

**Exemple 13 : Dépolymérisation de la subérine issu du liège avec le triéthylsilane ($Et_3SiH$)**

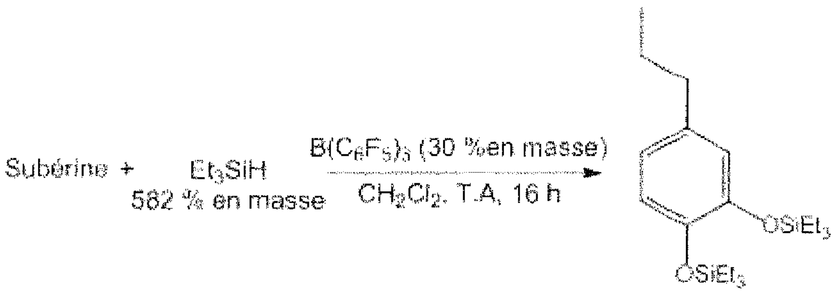


**IIf (12 % en masse)**

Afin de tester la possibilité de dépolymérisation de la subérine par hydrosilylation avec $B(C_6F_5)_3$, du liège issu des bouchons de bouteilles de vin a été finement broyé puis séché sous vide pendant la nuit. Ensuite, le même mode opératoire que celui de l'exemple 1 est utilisé pour la dépolymérisation de la subérine. Dans ce cas, (100,0 mg) de liège sont utilisés avec (582,4 mg, 5,0 mmol, 582 % en masse) de $Et_3SiH$, (30 mg, 0,59 mmol, 30 % en masse) de $B(C_6F_5)_3$ et 3 mL de $CH_2Cl_2$. (conditions non optimisées). Après 16 heures de réaction à température ambiante (20 ± 5°C), une grande quantité du solide initialement insoluble est solubilisée. L'analyse GC-MS du résidu réactionnel montre la présence d'un mélange complexe de plusieurs produits parmi lesquelles le produit **IIf** a pu être identifié et quantifié à 12 % en masse par rapport à la masse de liège initialement introduite.

**B) Dépolymérisation de polymères oxygénés en présence de $(Ph_3)C^+B(C_6F_5)_3^-$**

**Exemple 14 : Dépolymérisation de PC-BPA en utilisant $(Ph_3)C^+B(C_6F_5)_3^-$**

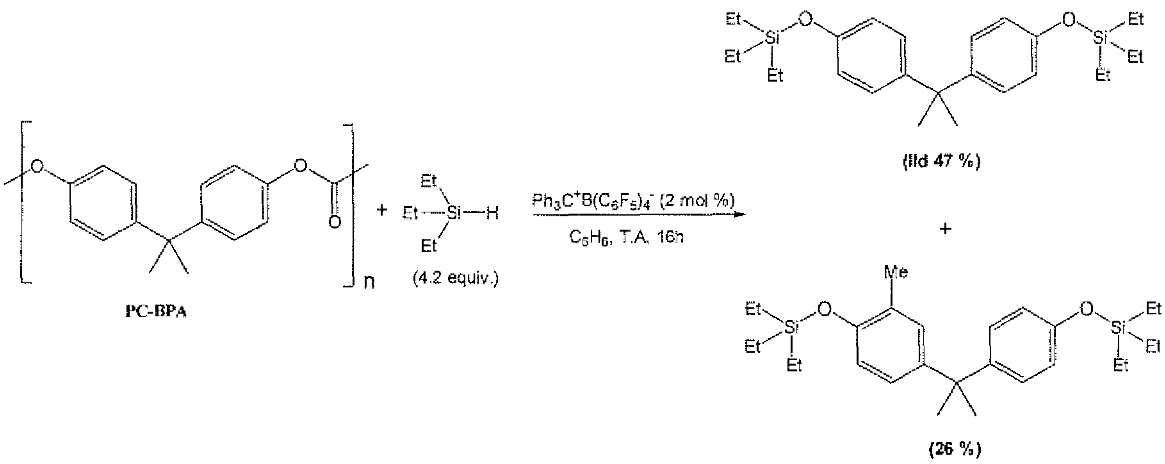

PC-BPA
(IId 47 %)
(26 %)

Le même mode opératoire que celui de l'exemple 1 est employé pour la dépolymérisation du PC-BPA en utilisant $(Ph_3)C^+B(C_6F_5)_3^-$. Dans ce cas (123,2 mg, 0,5 mmol, 1 équiv.) de PC-BPA sont utilisés avec (244,2 mg, 2,1 mmol, 4,2 équiv.) de triéthylsilane et (9,2 mg, 0,01 mmol, 2 mol%) de $(Ph_3)C^+B(C_6F_5)_3^-$ dans $C_6H_6$ (3 mL). Après 16 heures de réaction, la conversion est totale et **IId** est obtenu avec 47 % de rendement. La purification des produit se réalise en suivant le même mode opératoire décrit dans l'exemple 1.

**C) Dépolymérisation de polymères oxygénés en présence du catalyseur d'iridium ($[(POCOP)Ir(H)(acétone)]^+B(C_6F_5)_4^-$)**

**Exemple 15 : Dépolymérisation du PET en utilisant ($[(POCOP)Ir(H)(acétone)]^+$ $B(C_6F_5)_4^-$)**

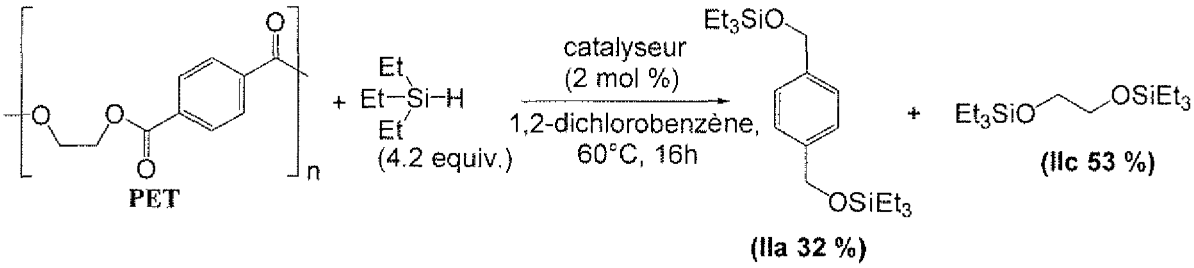

PET
(IIc 53 %)
(IIa 32 %)

catalyseur =

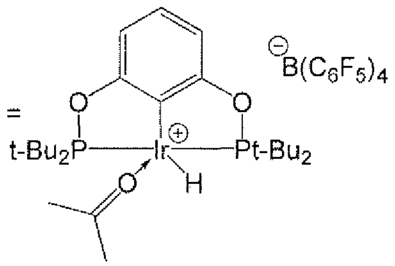

Le même mode opératoire employé pour la dépolymérisation du PET par $Et_3SiH$ et $B(C_6F_5)_3$ est employé pour la dépolymérisation du PET par $Et_3SiH$ et le catalyseur d'iridium ($[(POCOP)Ir(H)(acétone)]^+B(C_6F_5)_4^-$). Dans ce cas, (96,1 mg, 0,5 mmol, 1 équiv.) de PET sont utilisés avec (291,1 mg, 2,5 mmol, 5 équiv.) de triéthylsilane, 1,5 mL de 1,2-dichlorobenzène et (13.4 mg, 0,01 mmol, 2 mol%) de ($[(POCOP)Ir(H)(acétone)]^+B(C_6F_5)_4^-$) Après 16 heures de réaction à 60 ± 5°C les produits **IIa** et **IIc** sont produits avec des rendements respectifs de 32 et 53 %. La purification des produits se réalise en suivant le même mode opératoire décrit dans l'exemple 2.

**Caractérisation des molécules obtenues**

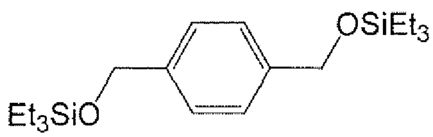

**IIa**

**$^1$H NMR** (200 MHz, $CDCl_3$, $Me_4Si$) δ (ppm) = 7.30 (4H, s, Ar-H), 4.72 (4H, s, $CH_2$-O), 1.05 - 0,91 (18 H, m, $CH_3CH_2Si$), 0.72 - 0.54 (12 H, m, $CH_3CH_2Si$).
**$^{13}$C NMR** (50 MHz, $CDCl_3$, $Me_4Si$): δ (ppm) = 140.2, 126.3, 64.7, 6.9, 4.6.
**MS:** IE (m/z): 337 (17); 205 (20); 154 (9); 118 (11); 117 (100); 115 (30); 112 (9); 105 (12); 104 (31); 103 (12); 87 (50); 75 (12); 59 (27).

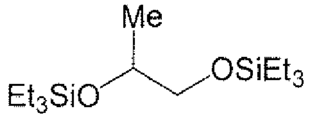

**IIb**

**$^1$H NMR** (200 MHz, $CDCl_3$, $Me_4Si$) δ (ppm) = 3.81 (1H, sex, $^3J$ = 6.1 Hz, Me-CH), 3.61 - 3.47 (1H, m, $CH_2$-O), 3.40 - 3.24 (1H, m, $CH_2$-O), 1.14 (3H, d, $^3J$ = 6.1 Hz, CH-$CH_3$), 1.04 - 0,88 (18 H, m, $CH_3CH_2Si$), 0.70 - 0.51 (12 H, m, $CH_3CH_2Si$).
**$^{13}$C NMR** (50 MHz, $CDCl_3$, $Me_4Si$): δ (ppm) = 69.3, 68.7, 20.9, 7.0, 6.9, 4.9, 4.5.
**MS:** IE (m/z): 275 (34); 217 (62); 189 (100); 161 (55); 159 (64); 133 (23); 131 (51); 115 (89); 105 (21); 95 (42); 87 (93); 81 (25); 59 (82).

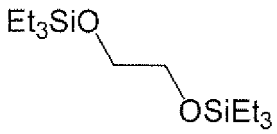

**IIc**

**$^1$H NMR** (200 MHz, $CDCl_3$, $Me_4Si$) δ (ppm) = 3.67 (4H, s, O-$CH_2$), 1.05 - 0,87 (18 H, m, $CH_3CH_2Si$), 0.70 - 0.52 (12 H, m, $CH_3CH_2Si$).
**$^{13}$C NMR** (50 MHz, $CDCl_3$, $Me_4Si$): δ (ppm) = 64.3, 6.9, 4.5.
**MS:** IE (m/z): 262 (11); 261 (44); 217 (20); 190 (13); 189 (66); 161 (28); 117 (11); 115 (65); 88 (37); 87 (100); 74 (14); 59 (56); 58 (12).

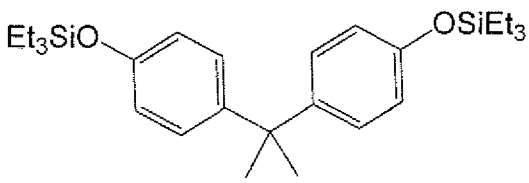

**IId**

**$^1$H NMR** (200 MHz, $CDCl_3$, $Me_4Si$) δ (ppm) = 6.95 (4H, d, $^3J$= 8.6 Hz, Ar-H), 6.62 (4H, d, $^3J$= 8.6 Hz, Ar-H), 1.51 (6H, s, $CH_3$-Cq), 0.97 - 0,79 (18 H, m, $CH_3CH_2Si$), 0.71 - 0.52 (12 H, m, $CH_3CH_2Si$).
**$^{13}$C NMR** (50 MHz, $CDCl_3$, $Me_4Si$): δ (ppm) = 153.3, 143.8, 127.8, 119.2, 41.4, 31.2, 6.8, 5.1.

**MS:** IE (m/z): 456 (13); 443 (14); 442 (37); 441 (59); 249 (22); 221 (11); 199 (17); 143 (13); 115 (15); 96 (12); 87 (100); 82 (10); 59 (52).

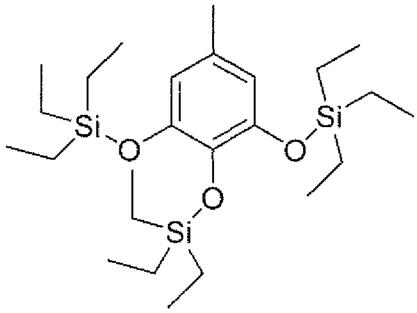

**IIe**

**$^1$H NMR** (200 MHz, $CDCl_3$, $Me_4Si$) δ (ppm) = 6.25 (2H, s, Ar-H), 2.16 (3H, s, Ar-$CH_3$), 1.10 - 0,85 (27 H, m, $CH_3CH_2Si$), 0.84 - 0.59 (18 H, m, $CH_3CH_2Si$).
**$^{13}$C NMR** (50 MHz, $CDCl_3$, $Me_4Si$): δ (ppm) = 147.9, 136.4, 129.6, 114.2, 21.3, 7.0, 6.8, 5.4, 5.2.
**MS:** IE (m/z): 483 (5); 310 (7); 309 (26); 147 (5); 116 (7); 115 (66); 88 (9); 87 (100); 86 (4); 60 (5); 59 (56); 58 (5); 32 (5).

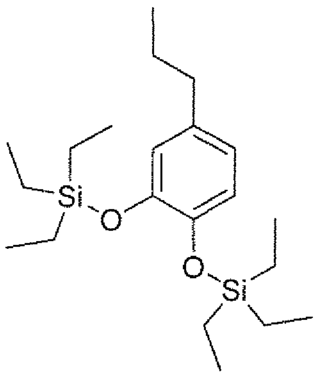

**IIf**

**$^1$H NMR** (200 MHz, $CDCl_3$, $Me_4Si$) δ (ppm) = 6.71 (1 H, d, $^3J$ = 8.1 Hz, Ar-H), 6.63 (1 H, s, Ar-H), 6.58 (1 H, d, $^3J$ = 8.1 Hz, Ar-H), 2.45 (2 H, t, $^3J$ = 7.8 Hz, Ar-$CH_2$), 1.57 (2 H, sex, $^3J$ = 7.8 Hz, $CH_2$-$CH_3$), 0.98 (18 H, t, $^3J$ = 7.9 Hz, $CH_3CH_2Si$), 0.90 (3 H, t, $^3J$ = 7.8 Hz, $CH_3CH_2Si$), 0.74 (12 H, q, $^3J$ = 7.9 Hz, $CH_3CH_2Si$).
**$^{13}$C NMR** (50 MHz, $CDCl_3$, $Me_4Si$): δ (ppm) =146.5, 144.7, 136.0, 121.3, 120.9, 120.2, 37.4, 24.7, 13.9, 6.9, 5.3, 5.2.
**MS:** IE (m/z): 380 (9); 351 (4); 207 (8); 117 (4) ; 116 (11); 115 (100); 88 (7); 87 (74); 59 (45); 58 (4).

**Exemple 16: Procédé de préparation de composés aromatiques comprenant une étape de dépolymérisation du PET en utilisant le triéthylsilane ($Et_3SiH$) / Recyclage d'un matériau plastique.**

Des déchets de bouteilles plastiques commerciales usagées de sodas sont collectées et les bouteilles réalisées en PET sont triées et isolées. Elles sont ensuite broyées et râpées manuellement en une poudre.
La poudre de PET est ensuite dépolymérisée selon les conditions de l'exemple 2.
Enfin, l'hydrolyse des produits **IIa** et **IIb** se réalise en agitant le mélange à 25°C pendant 2 heures dans une solution de TBAF.3$H_2O$ (2,1 équiv par rapport au nombre de moles de **IIa** et **IIb,** pris ensemble) dans le THF (3 mL). Les produits hydrolysés sont ensuite séparés par distillation sous pression réduite et purification sur colonne chromatographique. Cette procédure conduit à l'obtention de l'éthylène glycol (huile incolore, 72 % de rendement) et du 1,4-phénylènediméthanol (solide blanc, 85% de rendement).

**Abréviations utilisées :**

BHET = bis (hydroxyéthylène) téréphthalate
BPA=bisphénol A
DMC=dimeéthyl carbonate
EG= éthylène glycolPC-BPA = polymère carbonate du bisphénol APET = polyéthylène téréphthalate
PLA= acide polylactique
PLLA=Poly(L-lactide)

PS= polystyrène
PVC = chlorure de polyvinyle
TBAF= Fluorure de tétra-n-butylammonium
TBD=Triazabicyclodécène ou 1,5,7-Triazabicyclo[4.4.0]déc-5-èneTEG = triéthylène glycol
TPA= acide téréphtalique

## Revendications

**1.** Procédé de dépolymérisation de matériaux polymères oxygénés par clivage sélectif des liaisons oxygène-carbonyle des fonctions esters et des fonctions carbonates, **caractérisé en ce qu'**il comprend une étape de mise contact desdits matériaux polymères oxygénés avec un composé silane de formule (I)

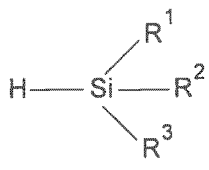

(I)

dans laquelle

- $R^1$, $R^2$ et $R^3$ représentent, indépendamment l'un de l'autre, un atome d'hydrogène, un atome d'halogène, un groupe hydroxyle, un groupe alkyle, un groupe alcényle, un groupe alcynyle, un groupe alkoxy, un groupe aryloxy, un groupe silylé, un groupe siloxy, un groupe aryle, un groupe amino, lesdits groupes alkyle, alcényle, alcynyle, alkoxy, silylé, siloxy, aryle et amino étant éventuellement substitués, ou
- $R^1$ est tel que défini ci-dessus et $R^2$ et $R^3$, pris ensemble avec l'atome de silicium auquel ils sont liés forment un hétérocycle silylé éventuellement substitué ;

en présence d'un catalyseur qui est :

- un catalyseur organique choisi parmi

  - les carbocations -choisis parmi le cation trityle ($(C_6H_5)_3C^+$), le tropilium $(C_7H_7)^+$, le cation benzylique ($C_6H_5CH_2^+$), le cation allylique ($CH_3$-$CH^+$-$CH$=$CH_2$), le méthylium ($CH_3^+$), le cyclopropylium ($C_3H_5^+$), le carbocation cyclopropylique choisi parmi le carbocation diméthyle cyclopropylique et le carbocation dicyclopropylique, le cation triazabicyclodécène (TBD), l'acylium $(R^1\text{-}C{=}O)^+$ avec $R^1$ choisi parmi le méthyle, le propyle et le benzyle, le benzénium $(C_6H_5)^+$, le cation norbornyle $(C_7H_{11})^+$ ;
  - les oxoniums choisis parmi $(CH_3)_3O^+BF_4^-$ et $(CH_3CH_2)_3O^+BF_4^-$ ;
  - un ion silylium choisi parmi $Et_3Si^+$ et $Me_3Si^+$ ;
  - les cations disilyles ayant un hydrure pontant choisis parmi les formules indiquées ci-dessous

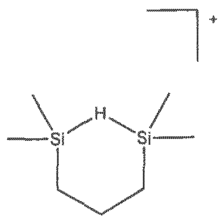

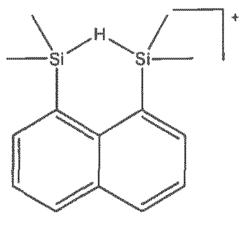

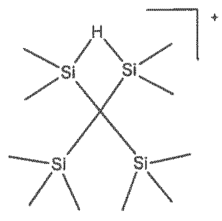

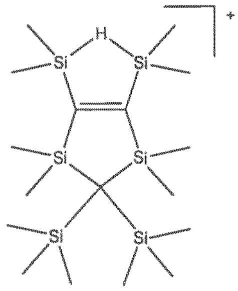

avec le contre ion anionique dudit ion silylium, desdits carbocations et desdits cations disilyles étant

- un halogénure choisi parmi $F^-$, $Cl^-$, $Br^-$ et $I^-$ ; ou
- un anion choisi parmi $BF_4^-$, $SbF_6^-$, $B(C_6F_5)_4^-$, $B(C_6H_5)_4^-$, $CF_3SO_3^-$, $PF_6^-$;

- un catalyseur organométallique choisi parmi :

- les complexes d'iridium de formule (II) :

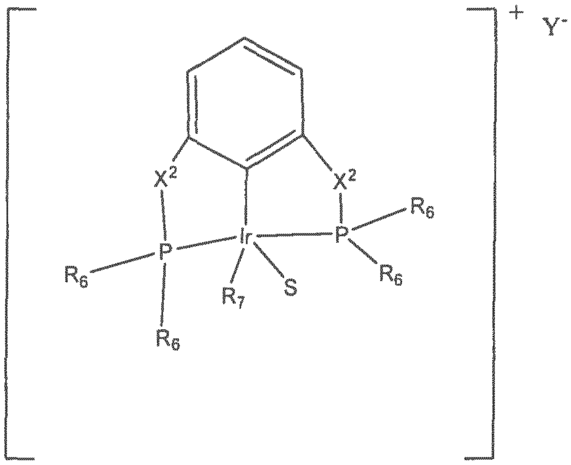

(II)

dans laquelle

- $R^6$ représente un groupe alkyle ou aryle ;
- $R^7$ représente un atome d'hydrogène ou un groupe alkyle ;
- $X^2$ représente un groupe $-CH_2-$ ou un atome d'oxygène ;
- $Y^-$ représente un contre ion choisi parmi $B(C_6F_5)_4^-$, et $B(C_6H_5)_4$ ; et
- S représente une molécule de solvant, coordonnée au complexe, choisi parmi le diméthylesulfoxyde (DMSO), l'acétonitrile ($CH_3CN$) et l'acétone ($CH_3COCH_3$) ; et

- les complexes de ruthénium de formule (III) :

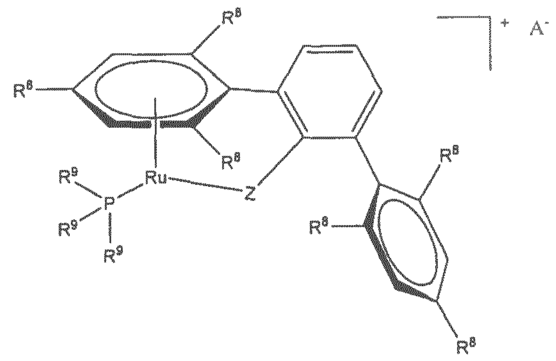

(III)

dans laquelle

- $R^8$ représente un atome d'hydrogène ou un groupe alkyle ;
- $R^9$ représente un aryle ou un groupe alkyle, lesdits groupes aryle et alkyle étant éventuellement substitués ;
- Z représente un groupe $-CH_2-$, un atome d'oxygène ou un atome de soufre ; et
- $A^-$ représente un contre ion choisi parmi $B(C_6F_5)_4^-$ et $[CHB_{11}H_5Cl_6]$ ; ou$^-$

- un catalyseur de type acide de Lewis choisi parmi :

▪ les composés de bore choisis parmi $BF_3$, $BF_3(Et_2O)$, $BCl_3$, $BBr_3$, le triphényle hydroborane, le tricyclohexyle hydroborane, $B(C_6F_5)_3$, le B-méthoxy-9-borabicyclo[3.3.1]nonane (B-méthoxy-9-BBN), le B-benzyl-9-borabicyclo[3.3.1]nonane (B-benzyl-9-BBN) ;
le composé borénium Me-TBD-BBN$^+$, les dérivés borenium ferrocène répondant à la formule

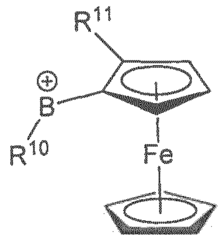

borenium ferrocène

dans laquelle $R^{10}$= phényle et $R^{11}$=3,5-diméthylpyridyle ;
▪ les composés de l'aluminium choisis parmi $AlCl_3$, $AlBr_3$, l'isopropoxyde d'aluminium (Al(O-i-Pr)$_3$), l'éthanoate d'aluminium ($Al(C_2H_3O_2)$), le sel de Krossing $[Ag(CH_2Cl_2)]\{Al[OC(CF_3)_3]_4\}$, le $Li\{Al[OC(CF_3)_3]_4\}$, $Et_2Al^+$ ;
▪ les composés d'indium choisis parmi $InCl_3$, $In(OTf)_3$ ;
▪ les composés de fer choisis parmi $FeCl_3$, $Fe(OTf)_3$ ;
▪ les composés de l'étain choisis parmi $SnCl_4$, $Sn(OTf)_2$ ;
▪ les composés du phosphore choisis parmi $PCl_3$, $PCl_5$, $POCl_3$ ;
▪ les composés trifluorométhanesulfonates ou triflates ($CF_3SO_3^-$) de métaux de transitions et des lanthanides choisis parmi le triflate de scandium, le triflate de ytterbium, le triflate d'yttrium, le triflate de cérium, le triflate de samarium, le triflate de niodinium, où OTf présente l'ion triflate ou trifluorométhanesulfonate de formule $CF_3SO_3^-$.

**2.** Procédé selon la revendication 1, **caractérisé en ce que** les polymères oxygénés sont choisis parmi

- les polyesters saturés ou insaturés choisis parmi l'acide polyglycolic (PGA), l'acide polylactique (PLA), le polycaprolactone (PCL), le polyhydroxyalkanoate (PHA), le polyhydroxybutyrate (P3HB), le polyhydroxyvalérate

(PHV), le polyéthylène adipate (PEA), le polybutylène succinate (PBS), le poly(3-hydroxybutyrate-*co*-3-hydroxyvalérate (PHBV), le polyéthylène téréphthalate (PET), le polybutylène téréphthalate (PBT), le polytriméthylène téréphthalate (PTT), le polyéthylène naphthalate (PEN),
- les polycarbonates choisis parmi le PC-BPA, le polypropylène carbonate (PPC), le polyéthylène carbonate (PEC), le poly(hexaméthylène carbonate), l'allyl diglycol carbonate (ADC) ou CR-39.
- les tannins hydrolysables notamment les gallotannins et les ellagitannins, et la subérine.

**3.** Procédé selon l'une des revendications 1 ou 2, **caractérisé en ce que** les polymères oxygénés sont choisis parmi

- le PET et le PLA ;
- le PC-BPA ;
- les gallotannins et les ellagitannins, et la subérine.

**4.** Procédé selon l'une quelconque des revendications 1 à 3, **caractérisé en ce que** le catalyseur organique est le triphénylcarbénium tetrakis(perfluorophenyl)borate $[(Ph)_3C^+B(C_6F_5)_4]^-$.

**5.** Procédé selon l'une quelconque des revendications 1 à 3, **caractérisé en ce que** le catalyseur organométallique est choisi parmi :

- le complexe d'iridium $[(POCOP)Ir(H)(acétone)]^+B(C_6F_5)_4^-$ avec (POCOP) représentant le 2,6-bis(di-tert-butylphosphinito)phényle ; et
- le complexe de ruthénium de formule (III) dans laquelle
  - $R^8$ représente un groupe méthyle ;
  - $R^9$ représente $p$-$FC_6H_4$ ;
  - Z représente un atome de soufre ; et
  - $A^-$ représente $B(C_6F_5)_4^-$.

**6.** Procédé selon l'une quelconque des revendications 1 à 3, **caractérisé en ce que** le catalyseur de type acide de Lewis est choisi parmi $BF_3$; $InCl_3$; le dérivé borenium ferrocène dans lequel $R^{10}$= phényle et $R^{11}$= 3,5-diméthylpyridyle.

**7.** Procédé selon l'une quelconque des revendications 1 à 6, **caractérisé en ce que** le composé silane mis en oeuvre est un composé silane de formule (I) dans laquelle $R^1$, $R^2$ et $R^3$ représentent, indépendamment l'un de l'autre, un atome d'hydrogène ; un groupe hydroxyle ; un groupe alkyle choisi parmi le méthyle, l'éthyle, le propyle, le butyle, et leurs isomères ramifiés ; un groupe alkoxy dont le radical alkyle est choisi parmi le méthyle, l'éthyle, le propyle, le butyle et leurs isomères ramifiés ; un groupe aryle choisi parmi le phényle et le benzyle ; un groupe aryloxy dont le radical aryle est choisi parmi le phényle et le benzyle ; un groupe siloxy $(-O-Si(X)_3)$ dont chaque X, indépendamment l'un de l'autre, est choisi parmi un atome d'hydrogène, un groupe alkyle choisi parmi le méthyle, l'éthyle, le propyle, un groupe aryle choisi parmi le phényle et le benzyle, un organosilane polymérique de formule générale

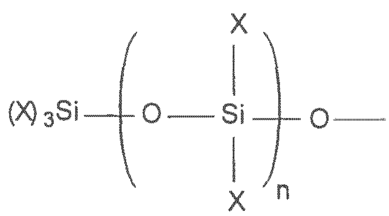

dans laquelle X est tel que défini ci-dessus et n est un nombre entier compris entre 1 et 20000, avantageusement entre 1 et 5000, plus avantageusement entre 1 et 1000 ; lesdits groupes alkyle, alkoxy, aryle, aryloxy, siloxy et aryle étant éventuellement substitués.

**8.** Procédé selon l'une quelconque des revendications 1 à 7, **caractérisé en ce que** le composé silane mis en oeuvre est un composé silane de formule (I) dans laquelle $R^1$, $R^2$ et $R^3$ représentent, indépendamment l'un de l'autre, un atome d'hydrogène ; un groupe alkyle choisi parmi le méthyle, l'éthyle, le propyle et son isomère ramifié ; un groupe aryle choisi parmi le benzyle et le phényle ; un groupe siloxy choisi parmi le polydiméthylsiloxane (PDMS), le polyméthylhydroxysiloxane (PMHS) et le tétraméthyldisiloxane (TMDS).

**9.** Procédé selon l'une quelconque des revendications 1 à 8, **caractérisé en ce que** le rapport molaire entre le composé silane de formule (I) et le matériau polymère oxygéné est compris entre 0,1 et 20, bornes incluses.

**10.** Procédé selon l'une quelconque des revendications 1 à 9, **caractérisé en ce que** la quantité de catalyseur est de 0,001 à 1 équivalent molaire, bornes incluses, par rapport au nombre de mole initial du matériau polymère oxygéné.

**11.** Procédé de recyclage de matériaux plastiques ou des mélanges de matériaux plastiques contenant au moins un polymère oxygéné **caractérisé en ce qu'**il comprend une étape de dépolymérisation de matériaux polymères oxygénés selon l'une quelconque des revendications 1 à 10.

**12.** Procédé de préparation des composés aromatiques mono-, di-, et/ou tri-cycliques dont chaque cycle est éventuellement mono-, di- et /ou tri-oxygénés **caractérisé en ce qu'**il comprend une étape de dépolymérisation de matériaux polymères oxygénés selon l'une quelconque des revendications 1 à 10.

## Patentansprüche

**1.** Verfahren zur Depolymerisierung von sauerstoffhaltigen Polymermaterialien durch selektive Spaltung der Sauerstoff-Carbonylverbindungen von den Esterfunktionen und von den Carbonatfunktionen, **dadurch gekennzeichnet, dass** es einen Schritt des Inkontaktbringens der sauerstoffhaltigen Polymermaterialien mit einer Silanverbindung mit der Formel (I) umfasst

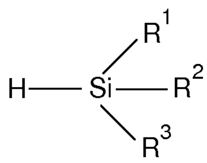

(I)

wobei

- $R^1$, $R^2$ und $R^3$, unabhängig voneinander, ein Wasserstoffatom, ein Halogenatom, eine Hydroxylgruppe, eine Alkylgruppe, eine Alkenylgruppe, eine Alkinylgruppe, eine Alkoxygruppe, eine Aryloxygruppe, eine Silylgruppe, eine Siloxygruppe, eine Arylgruppe, eine Aminogruppe darstellen, wobei die Alkyl-, Alkenyl-, Alkinyl-, Alkoxy-, Silyl-, Siloxy-, Aryl- und Aminogruppen eventuell substituiert sind, oder
- $R^1$ wie oben definiert ist und $R^2$ und $R^3$, zusammen mit dem Siliziumatom, an das sie gebunden sind, einen eventuell substituierten silylierten Heterocyclus bilden;

in Gegenwart eines Katalysators, der:

- ein organischer Katalysator, ausgewählt aus

  - den Carbokationen, ausgewählt aus dem Tritylkation ($(C_6H_5)_3C^+$), Tropylium $(C_7H_7)^+$, dem Benzylkation ($C_6H_5CH_2^+$), dem Allylkation ($CH_3$-$CH^+$-$CH$=$CH_2$), Methylium ($CH_3^+$), Cyclopropylium ($C_3H_5^+$), dem Cyclopropylcarbokation, ausgewählt aus dem Dimethyl-Cyclopropylcarbokation und dem Dicyclopropylcarbokation, dem Triazabicyclodecenkation (TBD), Acylium ($R^1$-C=O$)^+$ mit $R^1$, ausgewählt aus Methyl, Propyl und Benzyl, Benzenium $(C_6H_5)^+$, dem Norbornylkation $(C_7H_{11})^+$;
  - den Oxoniumionen, ausgewählt aus $(CH_3)_3O^+BF_4^-$ und $(CH_3CH_2)_3O^+BF_4^-$;
  - einem Silyliumion, ausgewählt aus $Et_3Si^+$ und $Me_3Si^+$;
  - wobei die Disilylkationen eine Hydridbrücke aufweisen, ausgewählt aus den unten angegebenen Formeln

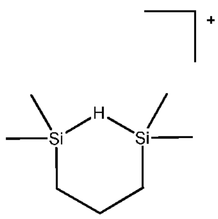
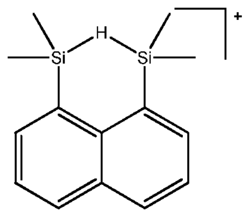
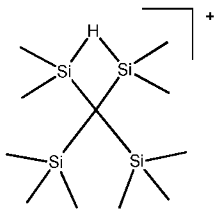

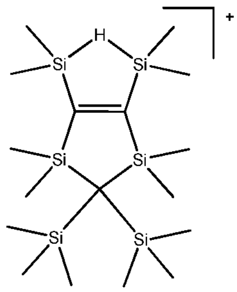

wobei das anionische Gegenion des Silyliumions, der Carbokationen und der Disilylkationen

• ein Halogenid, ausgewählt aus $F^-$, $Cl^-$, $Br^-$ und $I^-$; oder

• ein Anion, ausgewählt aus $BF_4^-$, $SbF_6^-$, $B(C_6F_5)_4^-$, $B(C_6H_5)_4^-$, $CF_3SO_3^-$, $PF_6^-$, ist;

- ein metallorganischer Katalysator, ausgewählt aus:

▪ den Iridiumkomplexen mit der Formel (II):

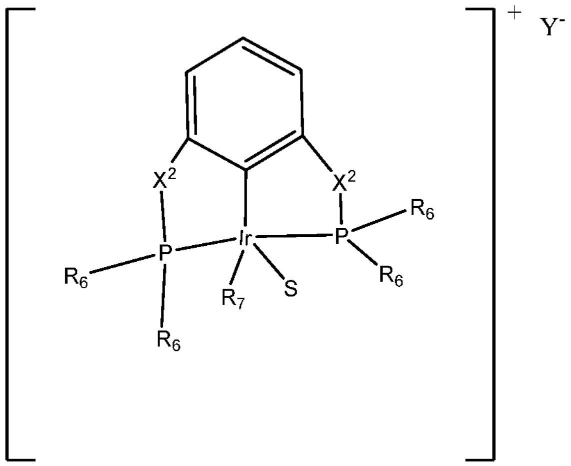

(II)

wobei

- $R^6$ eine Alkyl- oder Arylgruppe darstellt;
- $R^7$ ein Wasserstoffatom oder eine Alkylgruppe darstellt;
- $X^2$ eine $-CH_2-$ Gruppe oder ein Sauerstoffatom darstellt;
- $Y^-$ ein Gegenion, ausgewählt aus $B(C_6F_5)_4^-$ und $B(C_6H_5)_4$, darstellt; und
- S ein Lösemittelmolekül darstellt, das an den Komplex, ausgewählt aus Dimethylsulfoxid (DMSO), Acetonitril ($CH_3CN$) und Aceton ($CH_3COCH_3$), koordiniert ist; und

▪ den Rutheniumkomplexen mit der Formel (III):

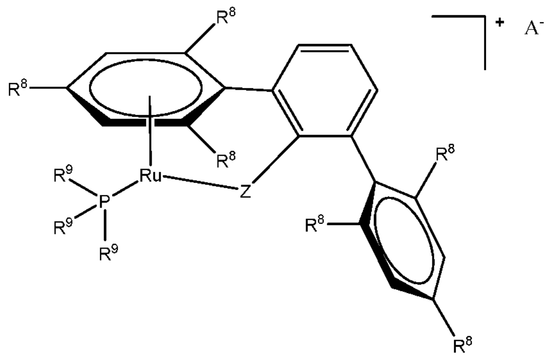

(III)

wobei

- $R^8$ ein Wasserstoffatom oder eine Alkylgruppe darstellt;
- $R^9$ Aryl oder eine Alkylgruppe darstellt, wobei die Aryl- und die Alkylgruppe eventuell substituiert sind;
- Z eine $-CH_2-$ Gruppe, ein Sauerstoffatom oder ein Schwefelatom darstellt; und
- $A^-$ ein Gegenion, ausgewählt aus $B(C_6F_5)_4^-$ und $[CHB_{11}H_5Cl_6]$, darstellt; oder$^-$
- ein Lewis-Säure-Katalysator ist, ausgewählt aus:

▪ den Borverbindungen, ausgewählt aus $BF_3$, $BF_3(Et_2O)$, $BCl_3$, $BBr_3$, Triphenyl-Hydroboran, Tricyclohexyl-Hydroboran, $B(C_6F_5)_3$, B-Methoxy-9-borabicyclo[3.3.1]nonan (B-Methoxy-9-BBN), B-Benzyl-9-borabicyclo[3.3.1]nonan (B-Benzyl-9-BBN);
der Boreniumverbindung Me-TBD-$BBN^+$, den Borenium-FerrocenDerivaten, die der folgenden Formel entsprechen

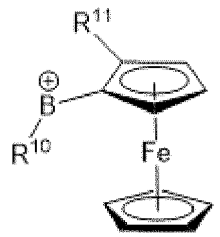

Borenium-Ferrocen

wobei $R^{10}$= Phenyl und $R^{11}$=3,5-Dimethylpyridyl;
▪ den Aluminiumverbindungen, ausgewählt aus $AlCl_3$, $AlBr_3$, Aluminiumisopropoxid (Al(O-i-Pr)$_3$), Aluminiumethanoat ($Al(C_2H_3O_2)$), dem Krossing-Salz $[Ag(CH_2Cl_2)]\{Al[OC(CF_3)_3]_4\}$, $Li\{Al[OC(CF_3)_3]_4\}$, $Et_2Al^+$;
▪ den Indiumverbindungen, ausgewählt aus $InCl_3$, $In(OTf)_3$;
▪ den Eisenverbindungen, ausgewählt aus $FeCl_3$, $Fe(OTf)_3$;
▪ den Zinnverbindungen, ausgewählt aus $SnCl_4$, $Sn(OTf)_2$;
▪ den Phosphorverbindungen, ausgewählt aus $PCl_3$, $PCl_5$, $POCl_3$;
▪ den Trifluoromethansulfonat- oder Triflatverbindungen ($CF_3SO_3^-$) von Übergangsmetallen und Lanthanoiden, ausgewählt aus Scandiumtriflat, Ytterbiumtriflat, Yttriumtriflat, Certriflat, Samariumtriflat, Neodymtriflat, wobei OTf das Triflat- oder Trifluoromethansulfonation mit der Formel $CF_3SO_3^-$ aufweist.

**2.** Verfahren nach Anspruch 1, **dadurch gekennzeichnet, dass** die sauerstoffhaltigen Polymere ausgewählt sind aus

- gesättigten oder ungesättigten Polyestern, ausgewählt aus Polyglycolsäure (PGA), Polymilchsäure (PLA), Polycaprolacton (PCL), Polyhydroxyalkanoat (PHA), Polyhydroxybutyrat (P3HB), Polyhydroxyvalerat (PHV), Polyethylenadipat (PEA), Polybutylensuccinat (PBS), Poly(3-hydroxybutyrat-*co*-3-hydroxyvalerat (PHBV), Polyethylenterephthalat (PET), Polybutylenterephthalat (PBT), Polytrimethylenterephthalat (PTT), Polyethylennaphthalat (PEN),
- Polycarbonaten, ausgewählt aus PC-BPA, Polypropylencarbonat (PPC), Polyethylencarbonat (PEC), Poly(hexamethylencarbonat), Allyldiglykolcarbonat (ADC) oder CR-39.
- hydrolysierbaren Tanninen, insbesondere Gallotanninen und Ellagitanninen und Suberin.

**3.** Verfahren nach einem der Ansprüche 1 oder 2, **dadurch gekennzeichnet, dass** die sauerstoffhaltigen Polymere ausgewählt sind aus

- PET und PLA;
- PC-BPA;
- Gallotanninen und Ellagitanninen und Suberin.

**4.** Verfahren nach einem der Ansprüche 1 bis 3, **dadurch gekennzeichnet, dass** der organische Katalysator Triphenylcarbeniumtetrakis(perfluorophenyl)borat $[(Ph)_3C^+B(C_6F_5)_4]^-$ ist.

**5.** Verfahren nach einem der Ansprüche 1 bis 3, **dadurch gekennzeichnet, dass** der metallorganische Katalysator ausgewählt ist aus:

- dem Iridiumkomplex $[(POCOP)Ir(H)(aceton)]^+B(C_6F_5)_4^-$, wobei (POCOP) 2,6-Bis(di-tert-butylphosphinito)phenyl darstellt; und
- dem Rutheniumkomplex mit der Formel (III), wobei
  - $R^8$ eine Methylgruppe darstellt;
  - $R^9$ *p*-$FC_6H_4$ darstellt;
  - Z ein Schwefelatom darstellt; und
  - $A^-$ $B(C_6F_5)_4^-$ darstellt.

**6.** Verfahren nach einem der Ansprüche 1 bis 3, **dadurch gekennzeichnet, dass** der Lewis-Säure-Katalysator ausgewählt ist aus $BF_3$; $InCl_3$; dem Borenium-Ferrocen-Derivat, wobei $R^{10}$= Phenyl und $R^{11}$= 3,5-Dimethylpyridyl.

**7.** Verfahren nach einem der Ansprüche 1 bis 6, **dadurch gekennzeichnet, dass** die eingesetzte Silanverbindung eine Silanverbindung mit der Formel (I) ist, wobei $R^1$, $R^2$ und $R^3$ unabhängig voneinander ein Wasserstoffatom; eine Hydroxylgruppe; eine Alkylgruppe, ausgewählt aus Methyl, Ethyl, Propyl, Butyl und deren verzweigten Isomeren; eine Alkoxygruppe, deren Alkylradikal ausgewählt ist aus Methyl, Ethyl, Propyl, Butyl und deren verzweigten Isomeren; eine Arylgruppe, ausgewählt aus Phenyl und Benzyl, eine Aryloxygruppe, deren Arylradikal ausgewählt ist aus Phenyl und Benzyl; eine Siloxygruppe $(-O-Si(X)_3)$ darstellen, wovon jedes X, unabhängig voneinander, ausgewählt ist aus einem Wasserstoffatom, einer Alkylgruppe, ausgewählt aus Methyl, Ethyl, Propyl, einer Arylgruppe, ausgewählt aus Phenyl und Benzyl, einem polymeren Organosilan mit der allgemeinen Formel

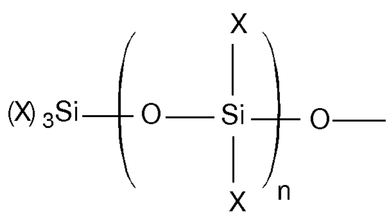

wobei X so wie oben definiert ist und n eine ganze Zahl zwischen 1 und 20000 ist, vorzugsweise zwischen 1 und 5000, bevorzugter zwischen 1 und 1000; wobei die Alkyl-, Alkoxy-, Aryl-, Aryloxy-, Siloxy- und Arylgruppen eventuell substituiert sind.

**8.** Verfahren nach einem der Ansprüche 1 bis 7, **dadurch gekennzeichnet, dass** die eingesetzte Silanverbindung eine Silanverbindung mit der Formel (I) ist, wobei $R^1$, $R^2$ und $R^3$ unabhängig voneinander, ein Wasserstoffatom; eine Alkylgruppe, ausgewählt aus Methyl, Ethyl, Propyl und deren verzweigten Isomeren; eine Arylgruppe, ausgewählt aus Benzyl und Phenyl; eine Siloxygruppe, ausgewählt aus Polydimethylsiloxan (PDMS), Polymethylhydroxysiloxan (PMHS) und Tetramethyldisiloxan (TMDS), darstellen.

**9.** Verfahren nach einem der Ansprüche 1 bis 8, **dadurch gekennzeichnet, dass** das Molverhältnis zwischen der Silanverbindung mit der Formel (I) und dem sauerstoffhaltigen Polymermaterial zwischen 0,1 und 20 ist, Grenzen eingeschlossen.

**10.** Verfahren nach einem der Ansprüche 1 bis 9, **dadurch gekennzeichnet, dass** die Katalysatormenge 0,001 bis 1 Moläquivalent ist, Grenzwerte eingeschlossen, in Bezug auf die anfängliche Molzahl des sauerstoffhaltigen Polymermaterials.

**11.** Verfahren zum Recyceln von Kunststoffmaterialien oder der Kunststoffmaterialgemische, die zumindest ein sauerstoffhaltiges Polymer enthalten, **dadurch gekennzeichnet, dass** es einen Schritt der Depolymerisierung der sauerstoffhaltigen Polymermaterialien nach einem der Ansprüche 1 bis 10 umfasst.

**12.** Verfahren zur Herstellung der aromatischen mono-, di-, und/oder tricyclischen Verbindungen, von denen jeder Cyclus eventuell mono-, di- und/oder tri-sauerstoffhaltig ist, **dadurch gekennzeichnet, dass** es einen Schritt der Depolymerisierung der sauerstoffhaltigen Polymermaterialien nach einem der Ansprüche 1 bis 10 umfasst.

## Claims

**1.** Method for depolymerising oxygenated polymer materials through selective cleavage of the oxygen-carbonyl bonds of the ester functions and carbonate functions, **characterised in that** it comprises a step whereby said oxygenated polymer materials are put into contact with a silane compound of formula (I)

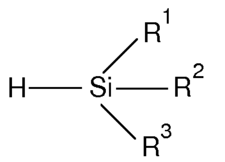

(I)

wherein

- $R^1$, $R^2$ and $R^3$ represent, independently from one another, a hydrogen atom, a halogen atom, a hydroxyl group, an alkyl group, an alkenyl group, an alkynyl group, an alkoxy group, an aryloxy group, a silyl group, a siloxy group, an aryl group, an amino group, where said alkyl, alkenyl, alkynyl, silyl, siloxy, aryl and amino groups can be optionally substituted, or
- $R^1$ is as defined above and $R^2$ and $R^3$, taken together with the silicon atom to which they are bonded, form a silyl heterocycle that can be optionally substituted;

in the presence of a catalyst, which is:

- an organic catalyst chosen from

  - Carbocations chosen from the trityl cation ($(C_6H_5)_3C^+$), tropylium $(C_7H_7)^+$, the benzylic cation ($C_6H_5CH_2^+$), the allyl cation ($CH_3$-$CH^+$-$CH$=$CH_2$), methenium ($CH_3^+$), cyclopropylium ($C_3H_5^+$), the cyclopropyl carbocation chosen from the cyclopropyl dimethyl carbocation and the dicyclopropyl carbocation, the triazabicyclodecene cation (TBD), acylium ($R^1$-C=O)$^+$ with $R^1$ chosen from methyl, propyl, benzyl, benzenium $(C_6H_5)^+$, the norbornyl cation $(C_7H_{11})^+$;
  - the oxonium chosen from $(CH_3)_3O^+BF_4^-$ and $(CH_3CH_2)_3O^+BF_4^-$;
  - a silylium ion chosen from $Et_3Si^+$ and $Me_3Si^+$;
  - the disilyl cations with a bridging hydride chosen from the formulae provided below

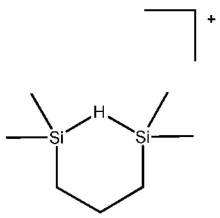
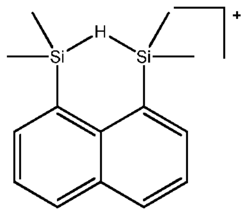
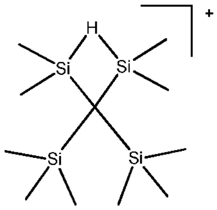

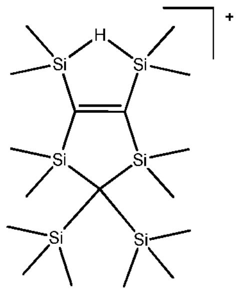

with the anionic counterion of said silylium ion, of said carbocations and of said disilyl cations being

- a halide chosen from $F^-$, $Cl^-$, $Br^-$ and $I^-$; or
- an anion chosen from $BF_4^-$, $SbF_6^-$, $B(C_6F_5)_4^-$, $B(C_6H_5)_4^-$, $CF_3SO_3^-$, $PF_6^-$;

- an organometallic catalyst chosen from:

- iridium complexes of the formula (II):

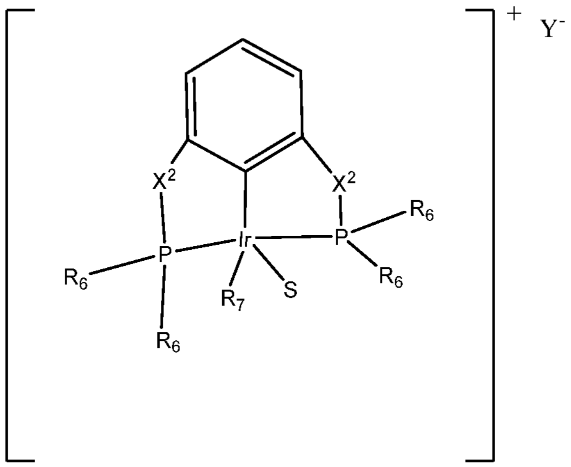

(II)

wherein

- $R^6$ represents an alkyl or aryl group;
- $R^7$ represents a hydrogen atom or an alkyl group;
- $X^2$ represents a $-CH_2-$ group or an oxygen atom;
- $Y^-$ represents a counterion chosen from $B(C_6F_5)_4^-$, and $B(C_6H_5)_4$; and
- S represents a solvent molecule coordinated with the complex, chosen from dimethyl sulfoxide (DMSO), acetonitrile ($CH_3CN$) and acetone ($CH_3COCH_3$); and

- ruthenium complexes of the formula (III):

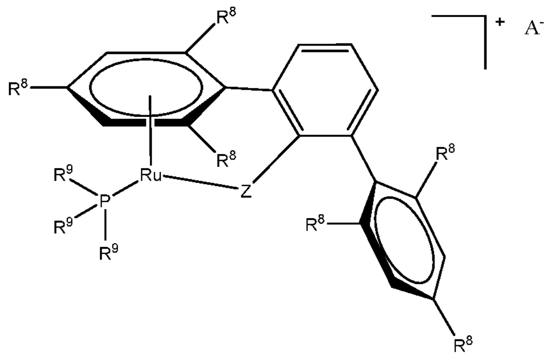

(III)

wherein

- $R^8$ represents a hydrogen atom or an alkyl group;
- $R^9$ represents an aryl or an alkyl group, where said aryl and alkyl groups can be substituted;
- Z represents a $-CH_2-$ group, an oxygen atom or a sulphur atom; and
- $A^-$ represents a counterion chosen from $B(C_6F_5)_4^-$ and $[CHB_{11}H_5Cl_6]$; or

- a catalyst of the Lewis acid type chosen from:

▪ boron compounds chosen from $BF_3$, $BF_3(Et_2O)$, $BCl_3$, $BBr_3$, triphenyl hydroborane, tricyclohexyl hydroborane, $B(C_6F_5)_3$, B-methoxy-9-borabicyclo[3.3.1]nonane (B-methoxy-9-BBN), B-benzyl-9-borabicyclo[3.3.1]nonane (B-benzyl-9-BBN);
the borenium compound Me-TBD-$BBN^+$, ferrocene borenium derivatives that follow the formula

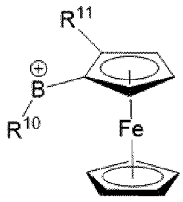

ferrocene borenium

wherein $R^{10}$= phenyl and $R^{11}$= 3,5-dimethylpyridyne;
▪ the aluminium compounds chosen from $AlCl_3$, $AlBr_3$, aluminium isopropoxide ($Al(O\text{-}i\text{-}Pr)_3$), aluminium ethanoate ($Al(C_2H_3O_2)$), Krossing salt $[Ag(CH_2Cl_2)]\{Al[OC(CF_3)_3]_4\}$, $Li\{Al[OC(CF_3)_3]_4\}$, $Et_2Al^+$ ;
▪ indium compounds chosen from $InCl_3$, $In(OTf)_3$;
▪ iron compounds chosen from $FeCl_3$, $Fe(OTf)_3$;
▪ tin compounds chosen from $SnCl_4$, $Sn(OTf)_2$;
▪ phosphorus compounds chosen from $PCl_3$, $PCl_5$, $POCl_3$;
▪ trifluoromethanesulfonate or triflate compounds ($CF_3SO_3^-$) of transition metals and lanthanides chosen from scandium triflate, ytterbium triflate, yttrium triflate, cerium triflate, samarium triflate, neodymium triflate where OTf presents the triflate or trifluoromethanesulfonate ion of the formula $CF_3SO_3^-$.

**2.** Method according to claim 1, **characterised in that** the oxygenated polymers are chosen from

- saturated or unsaturated polyesters chosen from polyglycolic acid (PGA), polylactic acid (PLA), polycaprolactone (PCL), le polyhydroxyalkanoate (PHA), polyhydroxybutyrate (P3HB), polyhydroxyvalerate (PHV), polyethylene adipate (PEA), polybutylene succinate (PBS), poly(3-hydroxybutyrate-*co*-3-hydroxyvalerate (PHBV), le polyethylene terephthalate (PET), polybutylene terephthalate (PBT), polytrimethylene terephthalate (PTT), polyethylene naphthalate (PEN),
- polycarbonates chosen from PC-BPA, polypropylene carbonate (PPC), polyethylene carbonate (PEC), poly(hexamethylene carbonate), allyl diglycol carbonate (ADC) or CR-39,

- hydrolyzable tannins and in particular gallotannins and ellagitannins, and suberin.

3. Method according to one of the claims 1 or 2, **characterised in that** the oxygenated polymers are chosen from

- PET and PLA;
- PC-BPA;
- gallotannins and ellagitannins, and suberin.

4. Method according to one of the claims 1 to 3, **characterised in that** the organic catalyst is triphenylcarbenium tetrakis(perfluorophenyl)borate $[(Ph)_3C^+B(C_6F_5)_4]^-$.

5. Method according to any of the claims 1 to 3, **characterised in that** the organometallic catalyst is chosen from:

- the iridium complex $[(POCOP)Ir(H)(acetone)]^+B(C_6F_5)_4^-$ with (POCOP) representing the 2,6-bis(di-tert-butylphosphinito)phenyl; and
- the ruthenium complex of the formula (III) wherein:
  - $R^8$ represents a methyl group;
  - $R^9$ represents *p*-$FC_6H_4$;
  - Z represents an atom of sulphur; and
  - $A^-$ represents $B(C_6F_5)_4^-$.

6. Method according to one of the claims 1 to 3, **characterised in that** the catalyst of the Lewis acid type is chosen from $BF_3$; $InCl_3$; the ferrocene borenium derivative wherein $R^{10}$= phenyl and $R^{11}$= 3,5-dimethylpyridyne.

7. Method according to one of the claims 1 to 6, **characterised in that** the implemented silane compound is a silane compound of formula (I) wherein $R^1$, $R^2$ et $R^3$ representing, independently from one another, an atom of hydrogen; a hydroxyl group; an alkyl group chosen from methyl, ethyl, propyl, butyl and their branched isomers; an alkoxy group of which the alkyl radical is chosen from methyl, ethyl, propyl, butyl and their branched isomers; an aryl group chosen from phenyl and benzyl; an aryloxy group of which the aryl radical is chosen from phenyl and benzyl; a siloxy group ($-O-Si(X)_3$) of which each X, independently from one another, is chosen from an atom of hydrogen, an alkyl group chosen from methyl, ethyl, propyl, aryl group chosen from phenyl and benzyl, a polymeric organosilane of a general formula

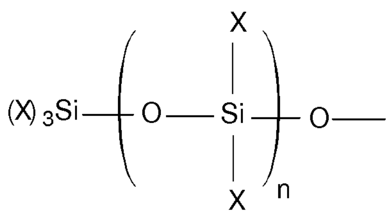

wherein X is as defined above, and n is an integer ranging from 1 to 20000, advantageously from 1 to 5000, more advantageously from 1 to 1000; said alkyl, alkoxy, aryl, aryloxy, siloxy and aryl group can be optionally substituted.

8. Method according to one of the claims 1 to 7, **characterised in that** the implemented silane compound is a silane compound of formula (I) wherein $R^1$, $R^2$ and $R^3$ represent, independently from one another, a hydrogen atom, an alkyl group chosen from methyl, ethyl, propyl and its branched isomer; an aryl group chosen from benzyl and phenyl; a siloxy group chosen from polydimethylsiloxane (PDMS), polymethylhydroxysiloxane (PMHS) and tetramethyldisiloxane (TMDS).

9. Method according to one of the claims 1 to 8, **characterised in that** the molar ratio between the silane compound of formula (I) and the oxygenated polymer material ranges from 0.1 to 20 inclusive.

10. Method according to one of the claims 1 to 9, **characterised in that** the quantity of catalyst is of 0.001 to 1 inclusive, molar equivalent, with respect to the initial number of moles of the oxygenated polymer material.

11. Method for recycling plastic materials or mixtures of plastic materials containing at least one oxygenated polymer,

**characterised in that** it comprises a step whereby oxygenated polymer materials are depolymerised according to one of the claims 1 to 10.

**12.** Method for preparing mono-, di-, and/or tri-cyclic aromatic compound, of which each cycle can be mono-, di- and/or tri-oxygenated, **characterised in that** it comprises a step whereby oxygenated polymer materials are depolymerised according to one of the claims 1 to 10.

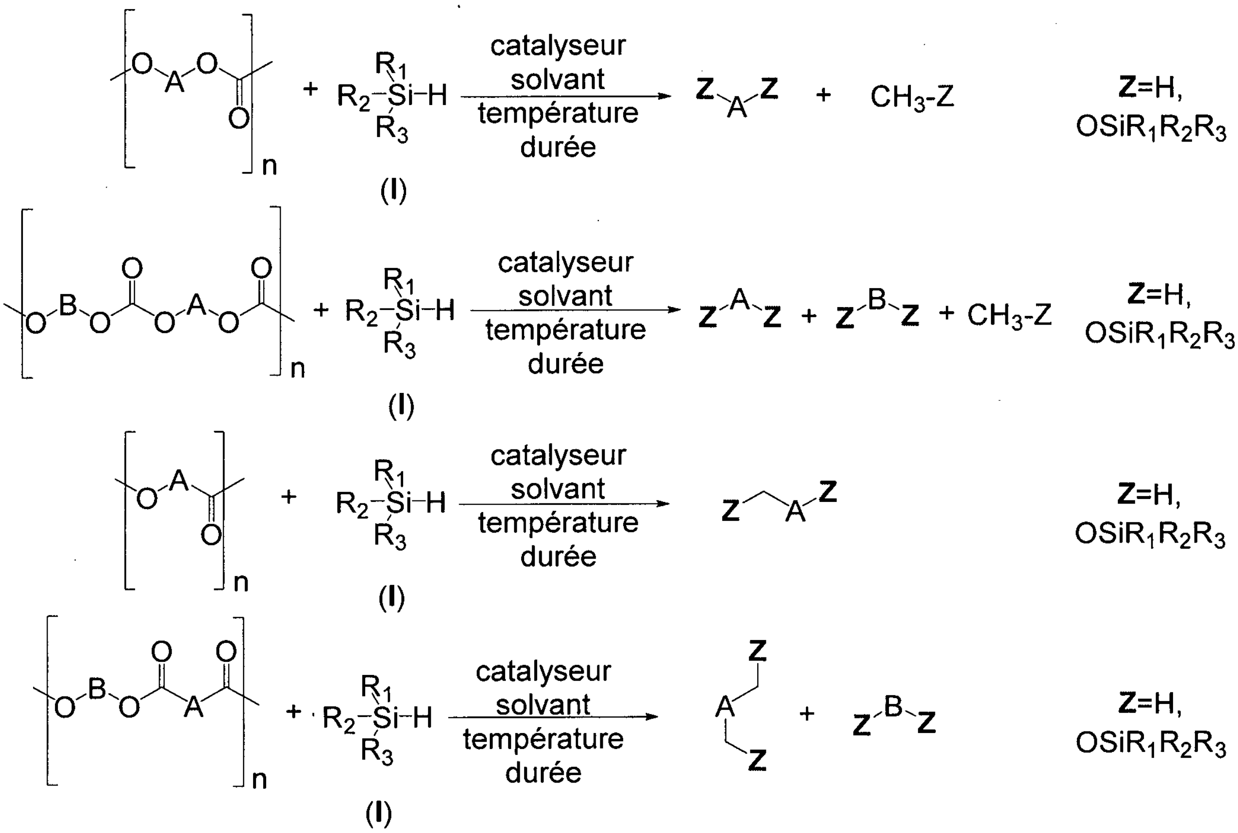
catalyseur
solvant
température
durée
(I)
Z=H,
OSiR1R2R3
catalyseur
solvant
température
durée
(I)
Z=H,
OSiR1R2R3
catalyseur
solvant
température
durée
(I)
Z=H,
OSiR1R2R3
catalyseur
solvant
température
durée
(I)
Z=H,
OSiR1R2R3

**Figure 1**

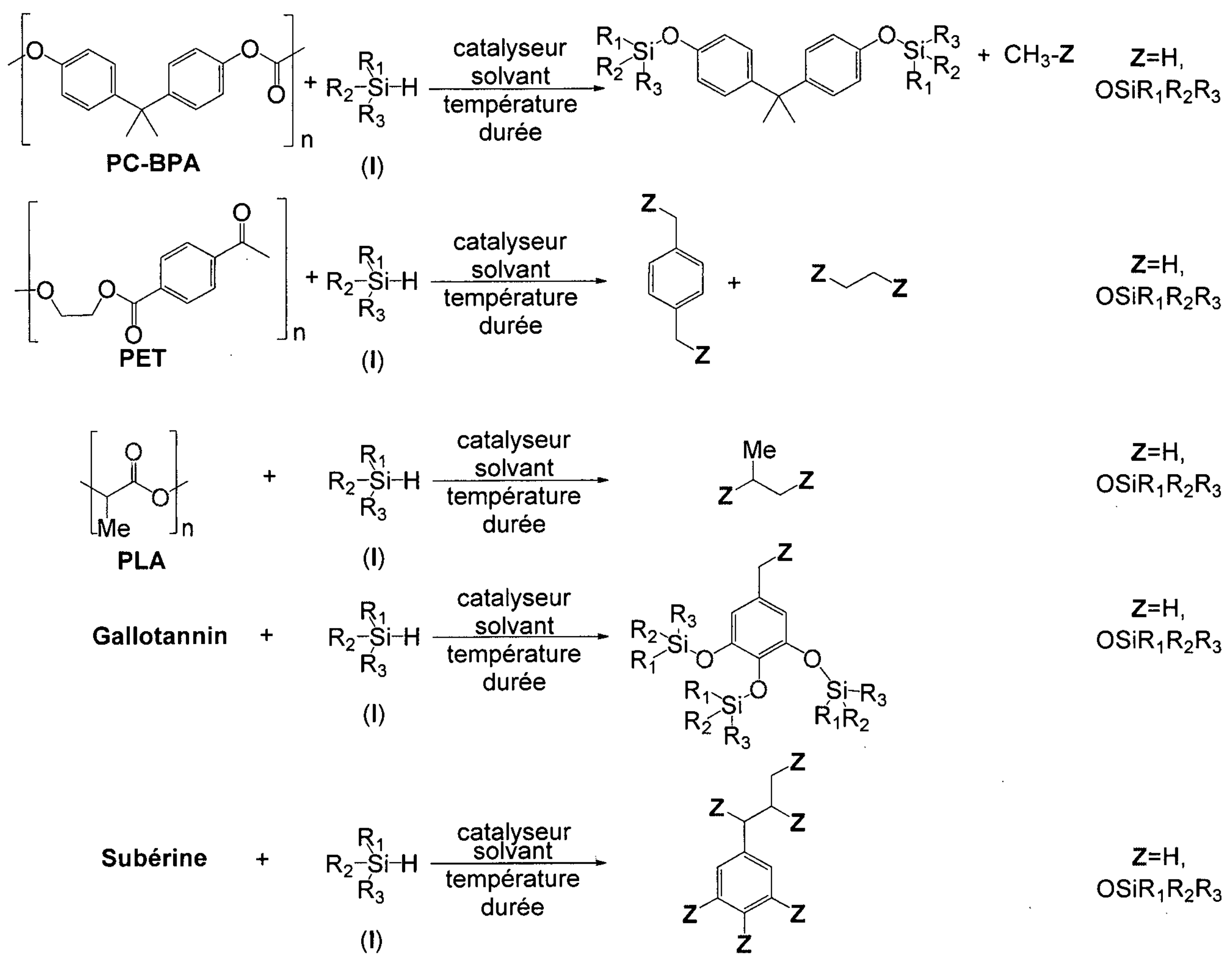
PC-BPA
(I)
catalyseur
solvant
température
durée
+ CH3-Z
Z=H,
OSiR1R2R3
PET
(I)
catalyseur
solvant
température
durée
Z=H,
OSiR1R2R3
PLA
(I)
catalyseur
solvant
température
durée
Z=H,
OSiR1R2R3
Gallotannin
(I)
catalyseur
solvant
température
durée
Z=H,
OSiR1R2R3
Subérine
(I)
catalyseur
solvant
température
durée
Z=H,
OSiR1R2R3

**Figure 2**

Hydrates de carbone de la paroi cellulaire

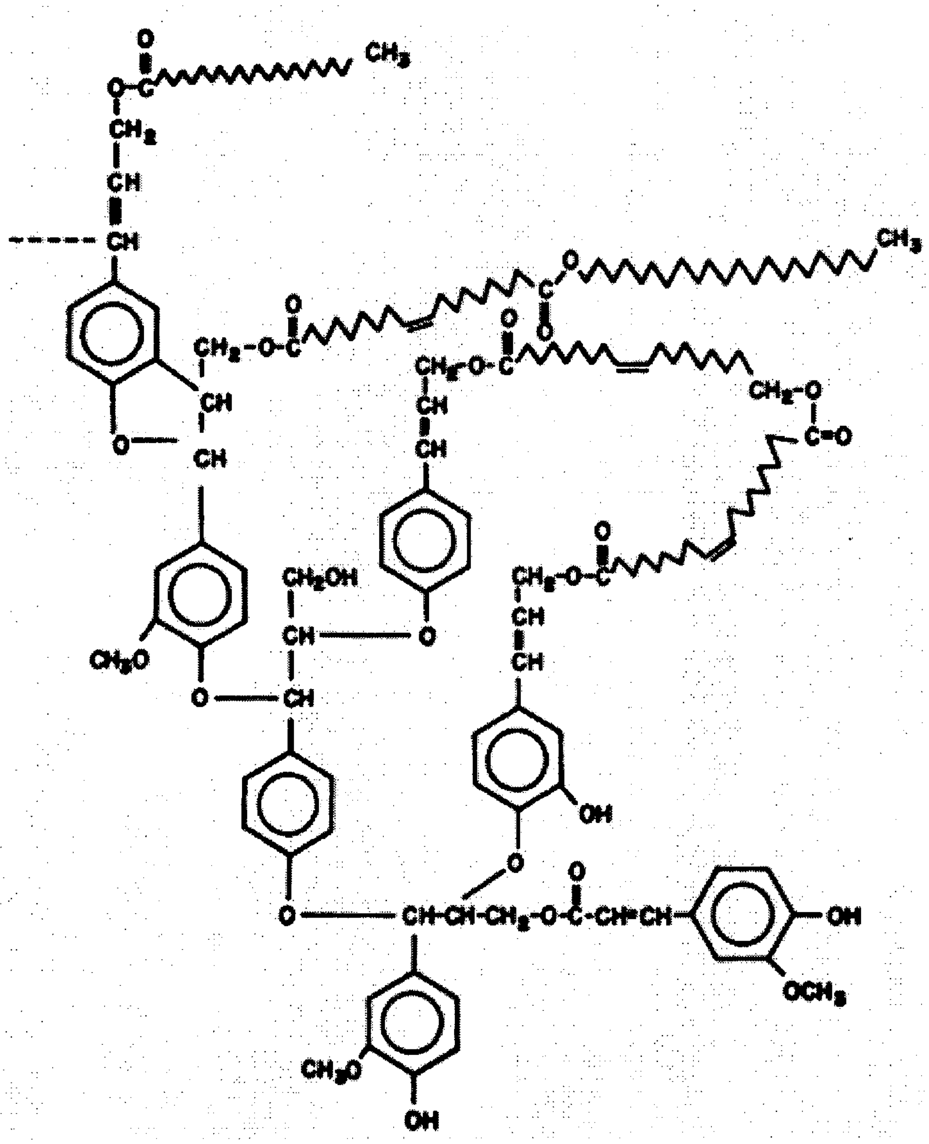

Figure 3

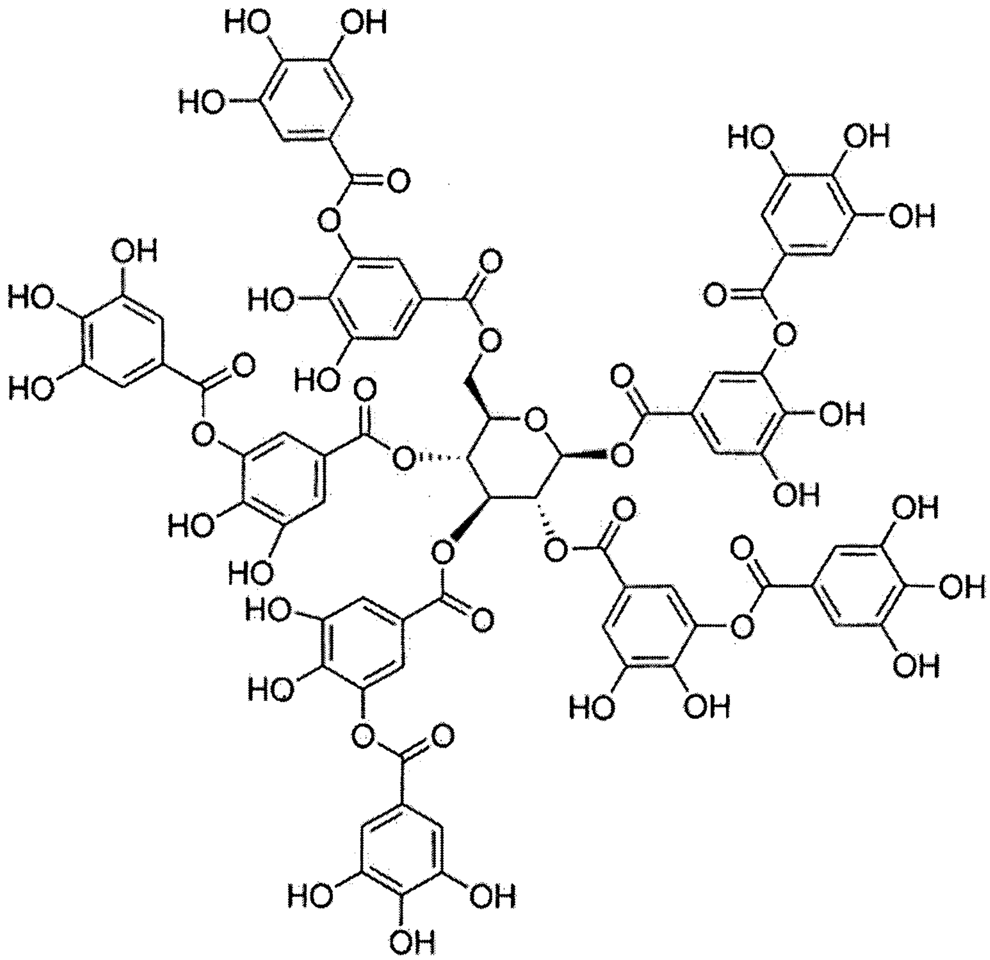

Figure 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **S.M. AL-SALEM ; P. LETTIERI ; J. BAEYENS.** *Progress in Energy and Combustion Science,* 2010, vol. 36, 103-129
- **S. H. PARK ; S. H. KIM.** *Fashion and Textiles,* 2014, vol. 1, 1-17
- **D. S. ACHILIAS ; D. A. LOUKA ; G. TSINTZOU ; I. A. KOUTSIDIS ; I. TSAGKALIAS ; L. ANDRIOTIS ; N. P. NIANIAS ; P. SIAFAKA.** Recent Advances in the Chemical Recycling of Polymers (PP, PS, LDPE, HDPE, PVC, PC, Nylon, PMMA). INTECH Open Access Publisher, 2012
- **E.M KRALL et al.** Controlled hydrogenative depolymerization of polyesters and polycarbonates catalyzed by ruthenium(ii) PNN pincer complexes. *CHEMICAL COMMUNICATIONS,* 01 Janvier 2014, vol. 50 (38), 4863-4960
- **C. S. NUNES et al.** PET depolymerisation in supercritical ethanol catalysed by [Bmim][BF4]. *RSC ADVANCES,* 15 Avril 2014, vol. 4 (39), 20308-20316
- **R. R. NAREDLA ; D. A. KLUMPP.** *Chem. Rev.,* 2013, vol. 113, 6905-6948
- **M. SAUNDERS ; H. A. JIMENEZ-VAZQUEZ.** *Chem. Rev.,* 1991, vol. 91, 375-397
- **I. GOTTKER-SCHNETMANN ; P. WHITE ; M. BROOKHART.** *J. Am. Chem. Soc.,* 2004, vol. 126, 1804-1811
- **J. YANG ; M. BROOKHART.** *J. Am. Chem. Soc.,* 2007, vol. 129, 12656-12657
- **T. STAHL ; H. F. T. KLARE ; M. OESTREICH.** *J. Am. Chem. Soc.,* 2013, vol. 135, 1248-1251
- **J. CHEN ; R. A. LALANCETTEA ; F. JÄKLE.** *Chem. Commun.,* 2013, vol. 49, 4893-4895
- **I. KROSSING.** *Chem.-Eur. J.,* 2001, vol. 7, 490
- **M. KHANDELWAL ; R. J. WEHMSCHULTE.** *Angew. Chem. Int. Ed.,* 2012, vol. 51, 7323-7326